# EUROPEAN PATENT SPECIFICATION

(11) **EP 3 319 138 B1**
(45) Date of publication and mention of the grant of the patent: **24.07.2019**
(21) Application number: 16817753.3
(22) Date of filing: 17.06.2016
(51) Int. Cl.: H01L 51/46, H01L 51/44, H01G 9/20

(54) **PHOTOELECTRIC CONVERSION ELEMENT, AND SOLAR BATTERY USING SAME**
FOTOELEKTRISCHES WANDLERELEMENT UND SOLARBATTERIE DAMIT
ÉLÉMENT DE CONVERSION PHOTOÉLECTRIQUE, ET CELLULE SOLAIRE L'UTILISANT

(30) Priority: 30.06.2015 JP 2015130772; 21.07.2015 JP 2015144422
(43) Date of publication of application: 09.05.2018
(73) Proprietor: FUJIFILM Corporation, Tokyo 106-8620 (JP)
(72) Inventor: SATOU, Hirotaka, Ashigarakami-gun Kanagawa 258-8577 (JP); HANAKI, Naoyuki, Ashigarakami-gun Kanagawa 258-8577 (JP); ISE, Toshihiro, Ashigarakami-gun Kanagawa 258-8577 (JP); SHIROKANE, Kenji, Ashigarakami-gun Kanagawa 258-8577 (JP)
(74) Representative: Hoffmann Eitle
(86) International application number: PCT/JP2016/068157
(87) International publication number: WO 2017/002645

(56) References cited:
- WO-A1-2014/189072
- CN-A- 103 664 995
- JP-A- 2014 175 472
- WEIJUN KE ET AL: "Perovskite Solar Cell with an Efficient TiO 2 Compact Film", ACS APPLIED MATERIALS & INTERFACES, vol. 6, no. 18, 28 August 2014 (2014-08-28), pages 15959-15965, XP055482700, US ISSN: 1944-8244, DOI: 10.1021/am503728d
- JIN HYUCK HEO ET AL: "Efficient inorganic-organic hybrid heterojunction solar cells containing perovskite compound and polymeric hole conductors", NATURE PHOTONICS, vol. 7, no. 6, 5 May 2013 (2013-05-05), pages 486-491, XP055097136, ISSN: 1749-4885, DOI: 10.1038/nphoton.2013.80
- JIN-DOU HUANG ET AL: "First-Principles Investigation of the Electronic and Conducting Properties of Oligothienoacenes and their Derivatives", CHEMISTRY - AN ASIAN JOURNAL, vol. 7, no. 5, 15 February 2012 (2012-02-15), pages 1032-1040, XP055342752, DE ISSN: 1861-4728, DOI: 10.1002/asia.201100904
- HIDEAKI EBATA ET AL: "Highly Soluble [1]Benzothieno[3,2- b ]benzothiophene (BTBT) Derivatives for High-Performance, Solution-Processed Organic Field-Effect Transistors", JOURNAL OF THE AMERICAN CHEMICAL SOCIETY, vol. 129, no. 51, 29 November 2007 (2007-11-29), pages 15732-15733, XP055250224, US ISSN: 0002-7863, DOI: 10.1021/ja074841i
- SH1, WEN ET AL.: 'Search for Organic Thermoelectric Materials with High Mobility: The Case of 2,7-Dialkyl[1]benzothieno[3,2-b][1] benzothiophene Derivatives' CHEMISTRY OF MATERIALS vol. 26, no. 8, 22 April 2014, pages 2669 - 2677, XP055342746
- ZHANG, SHOU-FENG ET AL.: 'Charge transport properties in a series of five-ring-fused thienoacenes: A quantum chemistry and molecular mechanic study' ORGANIC ELECTRONICS vol. 14, no. 2, February 2013, pages 607 - 620, XP028969045
- HUANG, JIN-DOU ET AL.: 'First-Principles Investigation of the Electronic and Conducting Properties of Oligothienoacenes and their Derivatives' CHEMISTRY AN ASIAN JOURNAL vol. 7, no. 5, 15 February 2012, pages 1032 - 1040, XP055342752

## Description

### FIELD

The present invention relates to a photoelectric conversion element, and a solar cell using the same.

### BACKGROUND

Photoelectric conversion elements are used in a variety of optical sensors, copiers, solar cells, and the like. It is expected that solar cells will be actively put into practical use as cells using non-exhaustible solar energy. Among these, research and development of dye sensitized solar cells, in which an organic dye, a Ru complex, or the like is used as a sensitizer, are actively in progress, and the photoelectric conversion efficiency thereof reaches approximately 11%.

Meanwhile, in recent years, there have been reported research results indicating that solar cells using a metal halide as a compound (perovskite compound) having a perovskite-type crystal structure are capable of achieving relatively high conversion efficiency, and the solar cells attract attention.

For example, Non-Patent Literature 1 reports that a perovskite-type solar cell, which uses a perovskite-type light absorbing agent in a photosensitive layer and which is provided with a hole transport layer and uses 2,2',7,7'-tetrakis-(N,N-di-p-methoxyphenylamine)9,9' -spirobifluorene [2,2',7,7'-tetrakis-(N,N-di-p-methoxyphenylamine)-9,9'-spirobifluorene: also referred to as "spiro-OMeTAD"] in the hole transport layer as a hole transporting material, can achieve high conversion efficiency.
Appl. Mater. Interfaces, 2014, 6, 15959-15965 and Nature Photonics, 2013, 6, 486-491 describe solar cells comprising perovskite type compounds.
Chemistry - An Asian Journal, 2012, 15, 1032-1040 and J. Am. Chem. Soc., 2007, 129, 15732-15733 describe oligothienoacene compounds.

### CITATION LIST

### [NON-PATENT LITERATURE]

[Non-Patent Literature 1] Science, 338, p. 643(2012)

### SUMMARY

### Technical Problem

However, according to an examination performed by the present inventors, the photoelectric conversion elements and the solar cell which are described in Non-Patent Literature 1 have a problem in that moisture resistance is low.

Accordingly, an object of the invention is to provide a photoelectric conversion element excellent in moisture resistance, and a solar cell using the same.

### Solution to Problem

The present inventors have made various examinations with respect to a photoelectric conversion element that includes a hole transport layer by using a perovskite-type light absorbing agent in a photosensitive layer, and found that a structure and properties of a hole transporting material that is used in the hole transport layer have an effect on moisture resistance of the photoelectric conversion element. The present inventors have made further examination, and as a result, they found that it is possible to improve the moisture resistance of the photoelectric conversion element in a case of using a hole transporting material having a specific structure. The invention has been accomplished on the basis of the finding.

That is, the above-described problem has been solved by the following means.
<1> A photoelectric conversion element comprises: a first electrode that includes a photosensitive layer, which includes a light absorbing agent, on a conductive support; and a second electrode that is opposite to the first electrode. The light absorbing agent includes a compound having a perovskite-type crystal structure that includes a cation of an element of Group 1 in the periodic table or a cationic organic group A, a cation of a metal atom M other than the element of Group 1 in the periodic table, and an anion of an anionic atom or atomic group X. A hole transport layer, which includes a hole transporting material, is provided between the first electrode and the second electrode. The hole transporting material includes at least one kind of compound represented by any one of Formula 1 to Formula 16, including a condensed polycyclic aromatic group having a number of rings of 4 or greater. At least two rings in the condensed polycyclic aromatic group are hetero rings including at least one atom selected from the group consisting of a sulfur atom, a nitrogen atom, a selenium atom, and an oxygen atom. The condensed polycyclic aromatic group includes at least one structure selected from the group consisting of a benzene ring, a naphthalene ring, an anthracene ring, and a phenanthrene ring as a partial structure 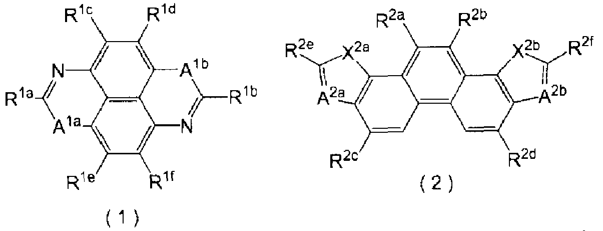 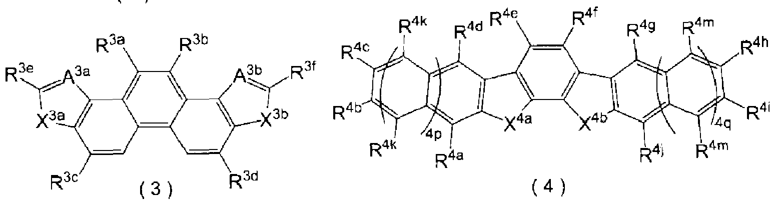 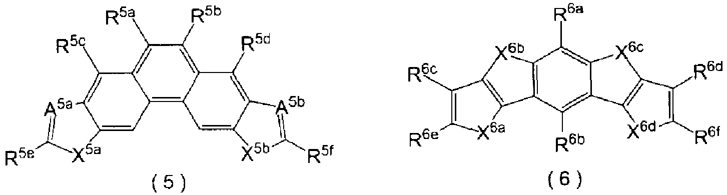 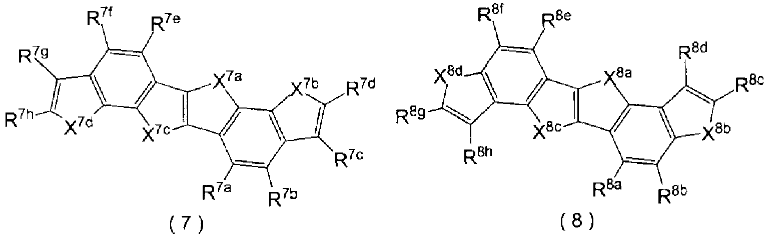 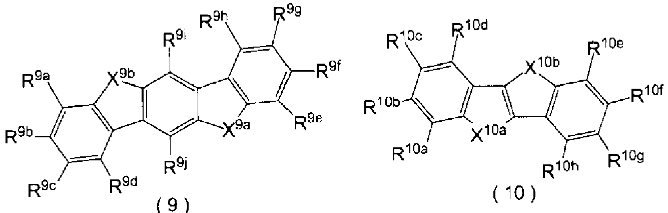 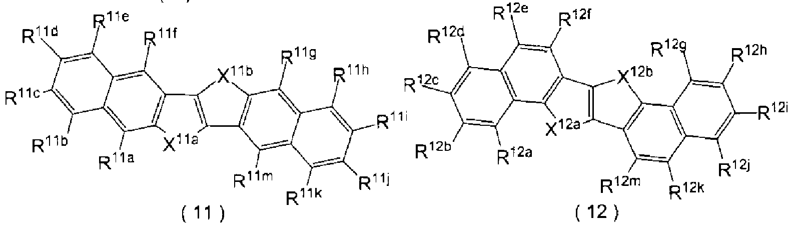 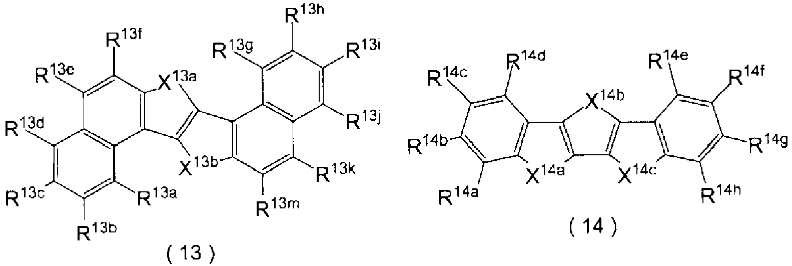 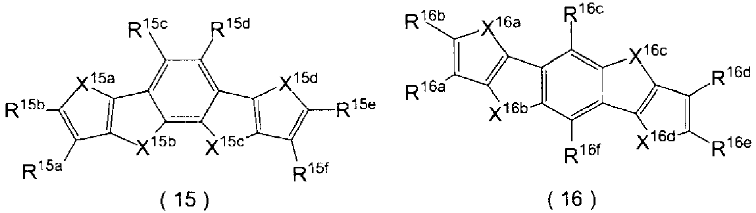
   in Formula 1, A^{1a} and A^{1b} each independently represent a S atom, an O atom, or a Se atom, and R^{1a} to R^{1f} each independently represent a hydrogen atom or a substituent group,
   in Formula 2, X^{2a} and X^{2b} each independently represent NR²ⁱ, an O atom, or a S atom, A^{2a} represents CR^{2g} or a N atom, A^{2b} represents CR^{2h} or a N atom, and R^{2a} to R²ⁱ each independently represent a hydrogen atom or a substituent group,
   in Formula 3, X^{3a} and X^{3b} each independently represent a S atom, an O atom, or NR^{3g}, A^{3a} and A^{3b} each independently represent CR^{3h} or a N atom, R^{3a} to R^{3h} each independently represent a hydrogen atom or a substituent group,
   in Formula 4, X^{4a} and X^{4b} each independently represent an O atom, a S atom, or a Se atom, 4p and 4q each independently represent an integer of 0 to 2, and R^{4a} to R^{4j}, R^{4k}, and R^{4m} each independently represent a hydrogen atom or a substituent group,
   in Formula 5, X^{5a} and X^{5b} each independently represent NR⁵ⁱ, an O atom, or a S atom, A^{5a} represents CR^{5g} or a N atom, A^{5b} represents CR^{5h} or a N atom, and R^{5a} to R⁵ⁱ each independently represent a hydrogen atom or a substituent group,
   in Formula 6, X^{6a} to X^{6d} each independently represent NR^{6g}, an O atom, or a S atom, and R^{6a} to R^{6g} each independently represent a hydrogen atom or a substituent group,
   in Formula 7, X^{7a} and X^{7c} each independently represent a S atom, an O atom, a Se atom, or NR⁷ⁱ, X^{7b} and X^{7d} each independently represent a S atom, an O atom, or a Se atom, and R^{7a} to R⁷ⁱ each independently represent a hydrogen atom or a substituent group,
   in Formula 8, X^{8a} and X^{8c} each independently represent a S atom, an O atom, a Se atom, or NR⁸ⁱ, X^{8b} and X^{8d} each independently represent a S atom, an O atom, or a Se atom, and R^{8a} to R⁸ⁱ each independently represent a hydrogen atom or a substituent group,
   in Formula 9, X^{9a} and X^{9b} each independently represent an O atom, a S atom, or a Se atom, and R^{9A} to R^{9j} each independently represent a hydrogen atom or a substituent group,
   in Formula 10, X^{10a} and X^{10b} each independently represent a S atom, an O atom, a Se atom, or NR¹⁰ⁱ, and R^{10a} to R¹⁰ⁱ each independently represent a hydrogen atom or a substituent group,
   in Formula 11, X^{11a} and X^{11b} each independently represent a S atom, an O atom, a Se atom, or NR¹¹ⁿ, and R^{11a} to R^{11k}, R^{11m}, and R¹¹ⁿ each independently represent a hydrogen atom or a substituent group,
   in Formula 12, X^{12a} and X^{12b} each independently represent a S atom, an O atom, a Se atom, or NR¹²ⁿ, and R^{12a} to R^{12k}, R^{12m}, and R¹²ⁿ each independently represent a hydrogen atom or a substituent group,
   in Formula 13, X^{13a} and X^{13b} each independently represent a S atom, an O atom, a Se atom, or NR¹³ⁿ, and R^{13a} to R^{13k}, R^{13m}, and R¹³ⁿ each independently represent a hydrogen atom or a substituent group,
   in Formula 14, X^{14a} to X^{14c} each independently represent a S atom, an O atom, a Se atom, or NR¹⁴ⁱ, and R^{14a} to R¹⁴ⁱ each independently represent a hydrogen atom or a substituent group,
   in Formula 15, X^{15a} to X^{15d} each independently represent a S atom, an O atom, a Se atom, or NR^{15g}, and R^{15a} to R^{15g} each independently represent a hydrogen atom or a substituent group, and
   in Formula 16, X^{16a} to X^{16d} each independently represent a S atom, an O atom, a Se atom, or NR^{16g}, and R^{16a} to R^{16g} each independently represent a hydrogen atom or a substituent group, and
   wherein at least one of R^{1a} to R^{1f} in Formula 1, at least one of R^{2a} to R²ⁱ in Formula 2, at least one of R^{3a} to R^{3h} in Formula 3, at least one of R^{4a} to R^{4j}, R^{4k}, and R^{4m} in Formula 4, at least one of R^{5a} to R⁵ⁱ in Formula 5, at least one of R^{6a} to R^{6g} in Formula 6, at least one of R^{7a} to R⁷ⁱ in Formula 7, at least one of R^{8a} to R⁸ⁱ in Formula 8, at least one of R^{9a} to R^{9j} in Formula 9, at least one of R^{10a} to R^{10h} in Formula 10, at least one of R^{11a} to R^{11k}, R^{11m}, and R¹¹ⁿ in Formula 11, at least one of R^{12a} to R^{12k}, R^{12m}, and R¹²ⁿ in Formula 12, at least one of R^{13a} to R^{13k}, R^{13m}, and R¹³ⁿ in Formula 13, at least one of R^{14a} to R¹⁴ⁱ in Formula 14, at least one of R^{15a} to R^{15g} in Formula 15, and at least one of R^{16a} to R^{16g} in Formula 16 are groups represented by the following Formula W,

      -L^{W}-R^{W} (W)

      in Formula W, L^{W} represents a divalent linking group represented by any one of the following Formulae L-1 to L-25, or a divalent linking group in which two or more divalent linking groups each being represented by any one of the following Formulae L-1 to L-25 are bonded to each other, and R^{W} represents a hydrogen atom or a substituent group, 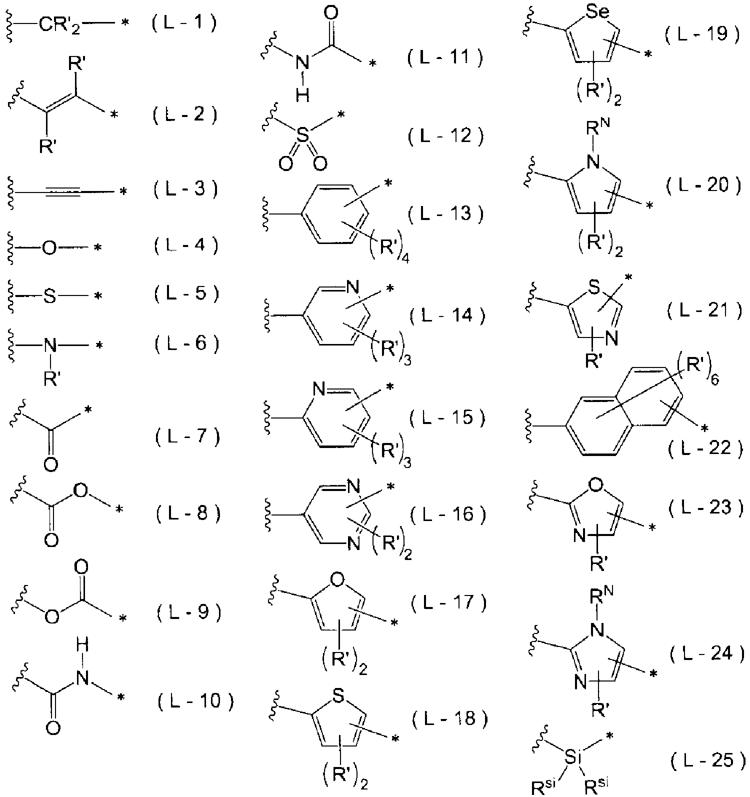 in Formulae L-1 to L-25, * represents a bonding position with R^{W}, a wavy-line portion represents another bonding position, R's in Formula L-1, Formula L-2, Formula L-6, and Formula L-13 to Formula L-24 each independently represent a hydrogen atom or a substituent group, R^{N}s represent a hydrogen atom or a substituent group, and R^{si}s each independently represent a hydrogen atom, an alkyl group, an alkenyl group, or an alkynyl group.
<2> In the photoelectric conversion element according to <1>, the hole transporting material may include at least one structure selected from the group consisting of a benzene ring, a naphthalene ring, and a phenanthrene ring as the partial structure.
<3> In the photoelectric conversion element according to <1> or <2>, the number of rings of the condensed polycyclic aromatic group may be 4 to 6.
<4> In the photoelectric conversion element according to any one of <1> to <3>, at least two hetero rings, which constitute the condensed polycyclic aromatic group, may respectively include one hetero atom.
<5> In the photoelectric conversion element according to any one of <1> to <4>, the hole transporting material may include at least one substituent group represented by the following Formula L-2', L-3', L-4', or L-6'. 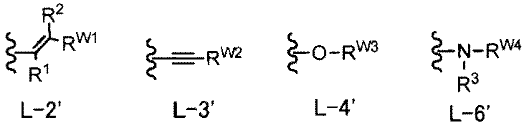
   In Formula, R¹ to R³, and R^{W1} to R^{W4} represent a hydrogen atom, an alkyl group, an aryl group, or a heteroaryl group.
<6> In the photoelectric conversion element according to <5>, the hole transporting material may include a substituent group that is represented by Formula L-2' or L-6'.
<7> In the photoelectric conversion element according to any one of <1> to <6>, the hole transporting material may include a linear alkyl portion having 8 or more carbon atoms, or a branched alkyl portion having 4 or more carbon atoms.
<8> A solar cell comprises the photoelectric conversion element according to any one of <1> to <7>.

### Advantageous Effects of Invention

According to the invention, since a hole transporting material having a specific structure is used, it is possible to provide a photoelectric conversion element excellent in moisture resistance and a solar cell using the photoelectric conversion element.

The above-described and other characteristics and advantages of the invention will be further clarified from the following description with appropriate reference to the accompanying drawings.

### BRIEF DESCRIPTION OF THE DRAWINGS

Fig. 1A is a cross-sectional view schematically illustrating a preferred aspect of a photoelectric conversion element of the invention.
Fig. 1B is an enlarged view of a circle portion in Fig. 1A.
Fig. 2 is a cross-sectional view schematically illustrating a preferred aspect including a thick photosensitive layer of the photoelectric conversion element of the invention.
Fig. 3 is a cross-sectional view schematically illustrating another preferred aspect of the photoelectric conversion element of the invention.
Fig. 4 is a cross-sectional view schematically illustrating still another preferred aspect of the photoelectric conversion element of the invention.

### DESCRIPTION OF EMBODIMENTS

### [Definition]

In this specification, parts of respective formulae may be expressed as a rational formula for understanding of chemical structures of compounds. According to this, in the respective formulae, partial structures are called (substituent) groups, ions, atoms, and the like, but in this specification, the partial structures may represent element groups or elements which constitute (substituent) groups or ions represented by the formulae in addition to the (substituent) groups, the ions, the atoms, and the like.

In this specification, with regard to expression of compounds (including a complex and a dye), the expression is also used to indicate salts of the compounds and ions of the compounds in addition to the compounds. In addition, the compounds include compounds of which a partial structure is changed in a range exhibiting a target effect. In addition, with regard to compounds for which substitution or non-substitution is not specified, the compounds also include compounds which have an arbitrary substituent group in a range exhibiting a desired effect. This is also true of substituent groups, linking groups, and the like (hereinafter, referred to as "substituent groups and the like").

In this specification, in a case where a plurality of substituent groups and the like expressed using specific symbols or a plurality of substituent groups and the like are simultaneously defined, the respective substituent groups and the like may be identical to or different from each other unless otherwise stated. This is also true of definition of the number of substituent groups and the like.

In addition, in a case of approaching each other (particularly, in a case of being close to each other), the plurality of substituent groups and the like may be bonded to each other to form a ring unless otherwise stated. In addition, rings, for example, alicycles, aromatic rings, and hetero rings may be additionally fused together to form a fused ring.

In this specification, numerical ranges represented by using "to" include ranges including numerical values before and after "to" as the lower limit and the upper limit.

### [Photoelectric Conversion Element]

The photoelectric conversion element of the invention includes a first electrode that includes a conductive support and a photosensitive layer provided on the conductive support, a second electrode that is opposite to the first electrode, and a hole transport layer that is provided between the first electrode and the second electrode.

In the invention, the aspect in which the photosensitive layer is provided on the conductive support includes an aspect in which the photosensitive layer is provided (directly provided) to be in contact with a surface of the conductive support, and an aspect in which the photosensitive layer is provided on an upper side of the surface of the conductive support through another layer.

In the aspect in which the photosensitive layer is provided on the upper side of the surface of the conductive support through another layer, as the other layer that is provided between the conductive support and the photosensitive layer, there is no particular limitation as long as the other layer does not deteriorate a battery performance of a solar cell. Examples of the other layer include a porous layer, a blocking layer, an electron transport layer, a hole transport layer, and the like.

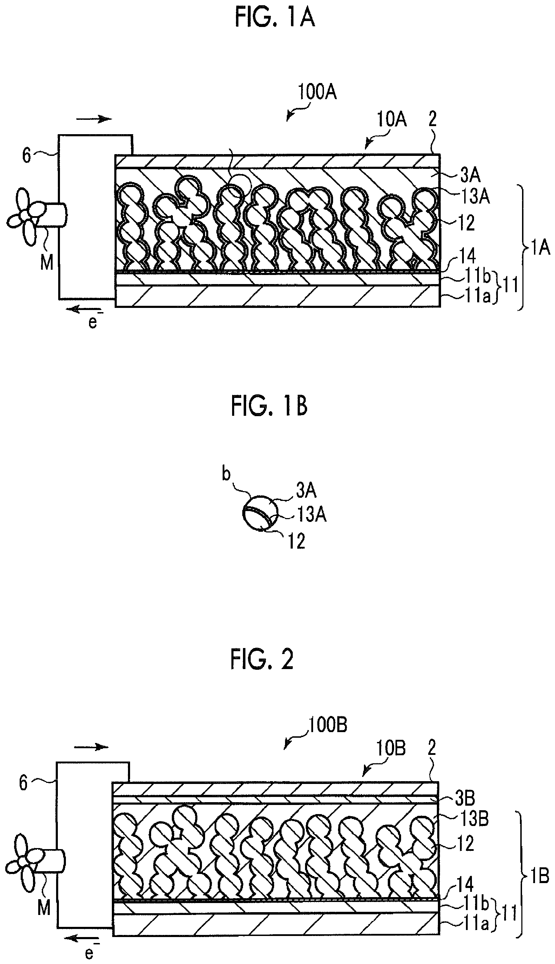

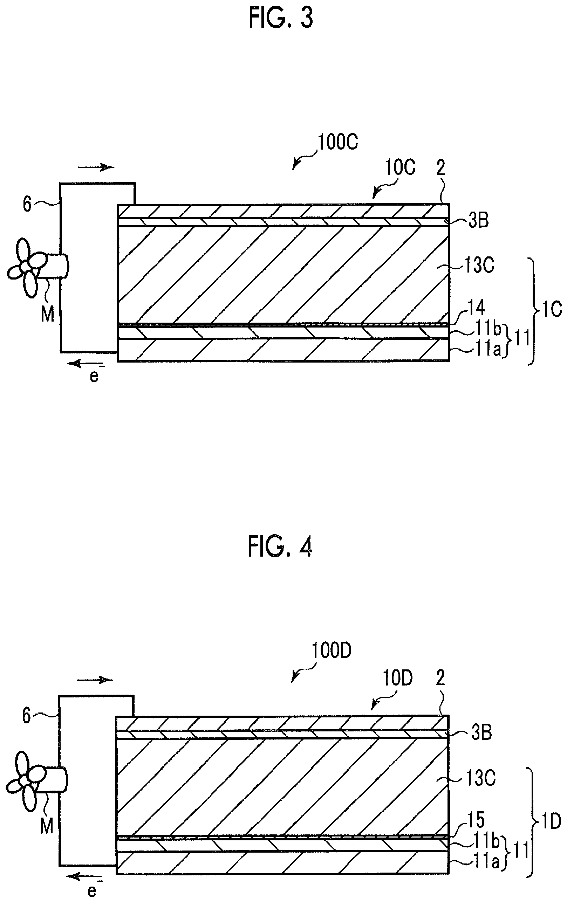

In the invention, examples of the aspect in which the photosensitive layer is provided on the upper side of the surface of the conductive support through another layer includes an aspect in which the photosensitive layer is provided on a surface of a porous layer in a thin film shape and the like (refer to Fig. 1A), an aspect in which the photosensitive layer is provided on the surface of the porous layer in a thick film shape (refer to Fig. 2), an aspect in which the photosensitive layer is provided on a surface of a blocking layer in a thick film shape (refer to Fig. 3), and an aspect in which the photosensitive layer is provided on a surface of an electron transport layer in a thick film shape (refer to Fig. 4). In addition, in Fig. 3, the photosensitive layer may be provided on the surface of the blocking layer in a thin film shape, and in Fig. 4, the photosensitive layer may be provided on the surface of the electron transport layer in a thin film shape. The photosensitive layer may be provided in a linear shape or in a dispersed pattern, but is preferably provided in a film shape.

In the photoelectric conversion element and a solar cell of the invention, a configuration other than a configuration defined in the invention is not particularly limited, and it is possible to employ a configuration that is known with respect to the photoelectric conversion element and the solar cell. Respective layers, which constitute the photoelectric conversion element of the invention, can be designed in correspondence with the purposes thereof, and may be formed, for example, in a monolayer or multilayers. For example, the porous layer may be provided between the conductive support and the photosensitive layer (refer to Fig. 1A and Fig. 2).

Hereinafter, description will be given of preferred aspects of the photoelectric conversion element of the invention.

In Fig. 1A, Fig. 1B, and Fig. 2 to Fig. 4, the same reference numeral represents the same constituent element (member).

Furthermore, in Fig. 1A and Fig. 2, the size of fine particles which form a porous layer 12 is illustrated in a highlighted manner. These fine particles are preferably packed with each other (are vapor-deposited or in close contact with each other) in the horizontal direction and the vertical direction with respect to a conductive support 11 to form a porous structure.

In this specification, simple description of "photoelectric conversion element 10" represents photoelectric conversion elements 10A to 10D unless otherwise stated. This is also true of a system 100 and a first electrode 1. In addition, simple description of "photosensitive layer 13" represents photosensitive layers 13A to 13C unless otherwise stated. Similarly, description of "hole transport layer 3" represents hole transport layers 3A and 3B unless otherwise stated.

Examples of a preferred aspect of the photoelectric conversion element of the invention include the photoelectric conversion element 10A illustrated in Fig. 1A. A system 100A illustrated in Fig. 1A is a system in which the photoelectric conversion element 10A is applied to a cell that allows operation means M (for example, an electric motor) to operate with an external circuit 6.

The photoelectric conversion element 10A includes a first electrode 1A, a second electrode 2, and a hole transport layer 3A.

The first electrode 1A includes a conductive support 11 including a support 11a and a transparent electrode 11b, a porous layer 12, and a photosensitive layer 13A including a perovskite-type light absorbing agent. As schematically illustrated in Fig. 1B in which a cross-sectional region b of Fig. 1A is enlarged, the photosensitive layer 13A is provided on a surface of the porous layer 12. In Fig. 1A, a blocking layer 14 is formed on the transparent electrode 11b, and the porous layer 12 is formed on the blocking layer 14. As described above, in the photoelectric conversion element 10A including the porous layer 12, it is assumed that a surface area of the photosensitive layer 13A increases, and thus charge separation and charge migration efficiency are improved.

The photoelectric conversion element 10B illustrated in Fig. 2 schematically illustrates a preferred aspect in which the photosensitive layer 13A of the photoelectric conversion element 10A illustrated in Fig. 1A is provided to be thick. In the photoelectric conversion element 10B, a hole transport layer 3B is provided to be thin. The photoelectric conversion element 10B is different from the photoelectric conversion element 10A illustrated in Fig. 1A in the film thickness of the photosensitive layer 13B and the hole transport layer 3B, but the photoelectric conversion element 10B has the same configuration as that of the photoelectric conversion element 10A except for the difference.

The photoelectric conversion element 10C illustrated in Fig. 3 schematically illustrates another preferred aspect of the photoelectric conversion element of the invention. The photoelectric conversion element 10C is different from the photoelectric conversion element 10B illustrated in Fig. 2 in that the porous layer 12 is not provided, but the photoelectric conversion element 10C has the same configuration as that of the photoelectric conversion element 10B except for the difference. That is, in the photoelectric conversion element 10C, the photosensitive layer 13C is formed on the surface of the blocking layer 14 in a thick film shape. In the photoelectric conversion element 10C, the hole transport layer 3B may be provided to be thick in the same manner as in the hole transport layer 3A.

The photoelectric conversion element 10D illustrated in Fig. 4 schematically illustrates still another preferred aspect of the photoelectric conversion element of the invention. The photoelectric conversion element 10D is different from the photoelectric conversion element 10C illustrated in Fig. 3 in that an electron transport layer 15 is provided instead of the blocking layer 14, but the photoelectric conversion element 10D has the same configuration as that of the photoelectric conversion element 10C except for the difference. The first electrode 1D includes the conductive support 11, and the electron transport layer 15 and the photosensitive layer 13C which are sequentially formed on the conductive support 11. The photoelectric conversion element 10D is preferable when considering that the respective layers can be formed from an organic material. According to this, the productivity of the photoelectric conversion element is improved, and thickness reduction or flexibilization becomes possible.

In the invention, a system 100 to which the photoelectric conversion element 10 is applied functions as a solar cell as follows.

Specifically, in the photoelectric conversion element 10, light that is transmitted through the conductive support 11 or the second electrode 2 and is incident to the photosensitive layer 13 excites a light absorbing agent. The excited light absorbing agent includes high-energy electrons and can emit the electrons. The light absorbing agent, which emits high-energy electrons, becomes an oxidized substance.

In the photoelectric conversion elements 10A to 10D, electrons emitted from the light absorbing agent migrate between a plurality of the light absorbing agents and reach the conductive support 11. The electrons which have reached the conductive support 11 work in the external circuit 6, and then return to the photosensitive layer 13 through the second electrode 2 and the hole transport layer 3. The light absorbing agent is reduced by the electrons which have returned to the photosensitive layer 13.

As described above, in the photoelectric conversion element 10, a cycle of excitation of the light absorbing agent and electron migration is repeated, and thus the system 100 functions as a solar cell.

In the photoelectric conversion elements 10A to 10D, a method of allowing an electron to flow from the photosensitive layer 13 to the conductive support 11 is different in correspondence with presence or absence of the porous layer 12, a kind thereof, and the like. In the photoelectric conversion element 10 of the invention, electron conduction, in which electrons migrate between the light absorbing agents, occurs. Accordingly, in a case where the porous layer 12 is provided, the porous layer 12 can be formed from an insulating substance other than semiconductors in the related art. In a case where the porous layer 12 is formed from a semiconductor, electron conduction, in which electrons migrate at the inside of semiconductor fine particles of the porous layer 12 or between the semiconductor fine particles, also occurs. On the other hand, in a case where the porous layer 12 is formed from an insulating substance, electron conduction in the porous layer 12 does not occur. In a case where the porous layer 12 is formed from the insulating substance, in a case of using fine particles of an aluminum oxide (Al₂O₃) as the fine particles of the insulating substance, a relatively high electromotive force (Voc) is obtained.

Even in a case where the blocking layer 14 as the other layer is formed from a conductor or a semiconductor, electron conduction in the blocking layer 14 occurs. In addition, even in the electron transport layer 15, electron conductor occurs.

In the invention, materials and respective members which are used in the photoelectric conversion element and the solar cell can be prepared by using a typical method except for materials and members which are defined in the invention. For example, with regard to a perovskite sensitized solar cell, for example, reference can be made to Non-Patent Literature 1 and J. Am. Chem. Soc., 2009, 131(17), p. 6050 to 6051.

In addition, reference can be made to materials and respective members which are used in a dye sensitized solar cell. With regard to dye sensitized solar cells, for example, reference can be made to JP2001-291534A, US4,927,721A, US4,684,537A, US5,0843,65A, US5,350,644A, US5,463,057A, US5,525,440A, JP1995-249790A (JP-H7-249790A), JP2004-220974A, and JP2008-135197A.

### [First Electrode]

The first electrode 1 includes the conductive support 11 and the photosensitive layer 13, and functions as a working electrode in the photoelectric conversion element 10.

As illustrated in Fig. 1A, and Fig. 2 to Fig. 4, it is preferable that the first electrode 1 includes at least one of the porous layer 12, the blocking layer 14, or the electron transport layer 15.

It is preferable that the first electrode 1 includes at least the blocking layer 14 from the viewpoint of short-circuit prevention, and more preferably the porous layer 12 and the blocking layer 14 from the viewpoints of light absorption efficiency and short-circuit prevention.

In addition, it is preferable that the first electrode 1 includes the electron transport layer 15 formed from an organic material from the viewpoints of an improvement in productivity of the photoelectric conversion element, thickness reduction, and flexibilization.

### [Conductive Support]

The conductive support 11 is not particularly limited as long as the conductive support 11 has conductivity and can support the photosensitive layer 13 and the like. It is preferable that the conductive support 11 has a configuration formed from a conductive material, for example, a metal, or a configuration including the support 11a formed from glass or plastic and the transparent electrode 11b formed on a surface of the support 11a as a conductive film.

Among these, as illustrated in Fig. 1A, and Fig. 2 to Fig. 4, it is more preferable that the conductive support 11 has a configuration in which a conductive metal oxide is applied to the surface of the support 11a formed from glass or plastic to form the transparent electrode 11b. Examples of the support 11a formed from plastic include a transparent polymer film described in Paragraph 0153 of JP2001-291534A. As a material that forms the support 11a, it is possible to use ceramic (JP2005-135902A) and a conductive resin (JP2001-160425A) in addition to glass or plastic. As a metal oxide, a tin oxide (TO) is preferable, and an indium-tin oxide (a tin-doped indium oxide; ITO) or a tin oxide doped with fluorine such as a fluorine-doped tin oxide (FTO) is more preferable. At this time, the amount of the metal oxide applied is preferably 0.1 to 100 g per square meter of a surface area of the support 11a. In a case of using the conductive support 11, it is preferable that light is incident from a support 11a side.

It is preferable that the conductive support 11 is substantially transparent. In the invention, "substantially transparent" represents that transmittance of light (having a wavelength of 300 to 1200 nm) is 10% or greater, preferably 50% or greater, and more preferably 80% or greater.

The thickness of the support 11a and the conductive support 11 is not particularly limited and is set to an appropriate thickness. For example, the thickness is preferably 0.01 µm to 10 mm, more preferably 0.1 µm to 5 mm, and still preferably 0.3 µm to 4 mm.

In a case of providing the transparent electrode 11b, the film thickness of the transparent electrode 11b is not particularly limited. For example, the film thickness is preferably 0.01 to 30 um, more preferably 0.03 to 25 µm, and still more preferably 0.05 to 20 µm.

The conductive support 11 or the support 11a may have a light management function on the surface. For example, the conductive support 11 or the support 11a may include an antireflection film formed by alternately laminating a high-refractive-index film and a low-refractive-index oxide film on the surface of the conductive support 11 or the support 11a as described in JP2003-123859A or may have a light guide function as described in JP2002-260746A.

### [Blocking Layer]

In the invention, as in the photoelectric conversion elements 10A to 10C, the blocking layer 14 is preferably provided on the surface of the transparent electrode 11b, that is, between the conductive support 11, and the porous layer 12, the photosensitive layer 13, the hole transport layer 3, or the like.

In the photoelectric conversion element and the solar cell, for example, in a case where the photosensitive layer 13 or the hole transport layer 3, and the transparent electrode 11b and the like are electrically connected to each other, a reverse current is generated. The blocking layer 14 plays a role of preventing the reverse current. The blocking layer 14 is also referred to as a "short-circuit prevention layer".

The blocking layer 14 may be allowed to function as a stage that carries the light absorbing agent.

The blocking layer 14 may be provided even in a case where the photoelectric conversion element includes the electron transport layer. For example, in a case of the photoelectric conversion element 10D, the blocking layer 14 may be provided between the conductive support 11 and the electron transport layer 15.

The material that forms the blocking layer 14 is not particularly limited as long as the material can perform the above-described function, and it is preferable that the material is a material through which visible light is transmitted, and which has insulating properties with respect to the conductive support 11 (transparent electrode 11b) and the like. Specifically, "material having insulating properties with respect to the conductive support 11 (transparent electrode 11b)" represents a compound (n-type semiconductor compound) having a conduction band energy level that is equal to or higher than a conduction band energy level of a material that forms the conductive support 11 (a metal oxide that forms the transparent electrode 11b) and is lower than a conduction band energy level of a material that constitutes the porous layer 12 or a ground state energy level of the light absorbing agent.

Examples of a material that forms the blocking layer 14 include silicon oxide, magnesium oxide, aluminum oxide, calcium carbonate, cesium carbonate, polyvinyl alcohol, polyurethane, and the like. In addition, the material may be a material that is typically used as a photoelectric conversion material, and examples thereof include titanium oxide, tin oxide, zinc oxide, niobium oxide, tungsten oxide, and the like. Among these, titanium oxide, tin oxide, magnesium oxide, aluminum oxide, and the like are preferred.

It is preferable that the film thickness of the blocking layer 14 is 0.001 to 10 µm, more preferably 0.005 to 1 µm, and still more preferably 0.01 to 0.1 µm.

In the invention, the film thicknesses of the respective layers can be measured by observing a cross-section of the photoelectric conversion element 10 by using a scanning electron microscope (SEM) and the like.

### [Porous Layer]

In the invention, as in the photoelectric conversion elements 10A and 10B, the porous layer 12 is preferably provided on the transparent electrode 11b. In a case where the blocking layer 14 is provided, the porous layer 12 is preferably formed on the blocking layer 14.

The porous layer 12 is a layer that functions as a stage that carries the photosensitive layer 13 on the surface. In a solar cell, so as to increase the light absorption efficiency, it is preferable to increase a surface area of at least a portion that receives light such as solar light, and it is preferable to increase the surface area of the porous layer 12 as a whole.

It is preferable that the porous layer 12 is a fine particle layer that includes pores and is formed through vapor deposition or close contact of fine particles of a material that forms the porous layer 12. The porous layer 12 may be a fine particle layer that is formed through vapor deposition of two or more kinds of fine particles. In a case where the porous layer 12 is a fine particle layer that includes pores, it is possible to increase the amount (adsorption amount) of the carried light absorbing agent.

It is preferable to increase the surface area of individual fine particles which constitute the porous layer 12 so as to increase the surface area of the porous layer 12. In the invention, in a state in which the fine particles are applied to the conductive support 11 and the like, it is preferable that the surface area of the fine particles which form the porous layer 12 is 10 or more times a projected area, and more preferably 100 or more times the projected area. The upper limit thereof is not particularly limited. Typically, the upper limit is approximately 5000 times the projected area. With regard to a particle size of the fine particles which form the porous layer 12, an average particle size, which uses a diameter when converting the projected area into a circle, is preferably 0.001 to 1 µm as primary particles. In a case where the porous layer 12 is formed by using a dispersion of fine particles, the average particle size of the fine particles is preferably 0.01 to 100 µm in terms of an average particle size of the dispersion.

For the material that forms the porous layer 12, there is no particular limitation with respect to conductivity. The material may be an insulating substance (insulating material), a conductive material, or a semiconductor (semi-conductive material).

As the material that forms the porous layer 12, it is possible to use, for example, chalcogenides (for example, an oxide, a sulfide, a selenide, and the like) of metals, compounds having a perovskite-type crystal structure (excluding a perovskite compound that uses a light absorbing agent), oxides of silicon (for example, silicon dioxide, and zeolite), or carbon nanotubes (including carbon nanowires, carbon nanorods, and the like).

The chalcogenides of a metal are not particularly limited, and preferred examples thereof include respective oxides of titanium, tin, zinc, tungsten, zirconium, hafnium, strontium, indium, cerium, yttrium, lanthanum, vanadium, niobium, aluminum, and tantalum, cadmium sulfide, cadmium selenide, and the like. Examples of the crystal structure of the chalcogenides of metals include an anatase-type crystal structure, a brookite-type crystal structure, and a rutile-type crystal structure, and the anatase-type crystal structure and the brookite-type crystal structure are preferable.

The compound having a perovskite-type crystal structure is not particularly limited, and examples thereof include a transition metal oxide and the like. Examples of the transition metal oxide include strontium titanate, calcium titanate, barium titanate, lead titanate, barium zirconate, barium stannate, lead zirconate, strontium zirconate, strontium tantalate, potassium niobate, bismuth ferrate, barium strontium titanate, lanthanum barium titanate, calcium titanate, sodium titanate, and bismuth titanate. Among these, strontium titanate, calcium titanate, and the like are preferable.

The carbon nanotubes have a shape obtained by rounding off a carbon film (graphene sheet) into a tubular shape.

The carbon nanotubes are classified into a single-walled carbon nanotube (SWCNT) obtained by winding one graphene sheet in a cylindrical shape, a double-walled carbon nanotube (DWCNT) obtained by winding two graphene sheets in a concentric shape, and a multi-walled carbon nanotube (MWCNT) obtained by winding a plurality of graphene sheets in a concentric shape. As the porous layer 12, any carbon nanotubes can be used without any particular limitation.

Among these, as the material that forms the porous layer 12, an oxide of titanium, tin, zinc, zirconium, aluminum, or silicon, or a carbon nanotube is preferable, and titanium oxide or aluminum oxide is more preferable.

The porous layer 12 may be formed from at least one kind of the chalcogenides of metals, the compound having a perovskite-type crystal structure, the oxide of silicon, or the carbon nanotube, or may be formed from a plurality of kinds thereof.

The film thickness of the porous layer 12 is not particularly limited. The thickness is typically in a range of 0.05 to 100 µm, and preferably in a range of 0.1 to 100 µm. In a case of being used as a solar cell, the film thickness is preferably 0.1 to 50 µm, and more preferably 0.2 to 30 µm.

The film thickness of the porous layer 12 can be measured by observing a cross-section of the photoelectric conversion element 10 with a scanning electron microscope (SEM).

Furthermore, the film thickness of other layers such as the blocking layer 14 can be measured in the same manner unless otherwise stated.

### [Electron Transport Layer]

In the invention, as in the photoelectric conversion element 10D, the electron transport layer 15 may be provided on the surface of the transparent electrode 11b.

The electron transport layer 15 has a function of transporting electrons, which are generated in the photosensitive layer 13, to the conductive support 11. The electron transport layer 15 is formed from an electron transporting material capable of exhibiting the above-described function. The electron transporting material is not particularly limited, and an organic material (organic electron transporting material) is preferable. Examples of the organic electron transporting material include fullerene compounds such as [6,6]-phenyl-C61-butyric acid methyl ester (PC₆₁BM), perylene compounds such as perylene tetracarboxylic diimide (PTCDI), low-molecular-weight compounds such as tetracyanoquinodimethane (TCNQ), high-molecular-weight compounds, and the like.

Although not particularly limited, it is preferable that the film thickness of the electron transport layer 15 is 0.001 to 10 µm, and more preferably 0.01 to 1 µm.

### [Photosensitive Layer (Light Absorbing Layer)]

The photosensitive layer 13 is preferably provided on the surface (including an inner surface of a concave portion in a case where a surface on which the photosensitive layer 13 is provided is uneven) of each of the porous layer 12 (in the photoelectric conversion elements 10A and 10B), the blocking layer 14 (in the photoelectric conversion element 10C), and the electron transport layer 15 (in the photoelectric conversion element 10D).

In the invention, the light absorbing agent may contain at least one kind of specific perovskite compound to be described later, or two or more kinds of perovskite compounds. In addition, the light absorbing agent may include a light absorbing agent other than the perovskite compound in combination with the perovskite compound. Examples of the light absorbing agent other than the perovskite compound include a metal complex dye, and an organic dye. At this time, a ratio between the perovskite compound and the light absorbing agent other than the perovskite compound is not particularly limited.

The photosensitive layer 13 may be a monolayer or a laminated layer of two or more layers. In a case where the photosensitive layer 13 has the laminated layer structure of two or more layers, the laminated layer structure may be a laminated layer structure obtained by laminating layers formed from light absorbing agents different from each other, or a laminated layer structure including an interlayer including a hole transporting material between a photosensitive layer and a photosensitive layer.

The aspect in which the photosensitive layer 13 is provided on the conductive support 11 is as described above. The photosensitive layer 13 is preferably provided on a surface of each of the layers in order for an excited electron to flow to the conductive support 11 or the second electrode 2. At this time, the photosensitive layer 13 may be provided on the entirety or a part of the surface of each of the layers.

The film thickness of the photosensitive layer 13 is appropriately set in correspondence with an aspect in which the photosensitive layer 13 is provided on the conductive support 11, and is not particularly limited. For example, the film thickness is preferably 0.001 to 100 µm, more preferably 0.01 to 10 µm, and still more preferably 0.01 to 5 µm.

In a case where the porous layer 12 is provided, a total film thickness including the film thickness of the porous layer 12 is preferably 0.01 µm or greater, more preferably 0.05 µm or greater, still more preferably 0.1 µm or greater, and still more preferably 0.3 µm or greater. In addition, the total film thickness is preferably 100 µm or less, more preferably 50 µm or less, and still more preferably 30 µm or less. The total film thickness may be set to a range in which the above-described values are appropriately combined. Here, as illustrated in Fig. 1A, in a case where the photosensitive layer 13 has a thin film shape, the film thickness of the photosensitive layer 13 represents a distance between an interface with the porous layer 12, and an interface with the hole transport layer 3 to be described later along a direction that is perpendicular to the surface of the porous layer 12.

In the photoelectric conversion element 10, a total film thickness of the porous layer 12, the photosensitive layer 13, and the hole transport layer 3 is not particularly limited. For example, the total thickness is preferably 0.01 µm or greater, more preferably 0.05 µm or greater, still more preferably 0.1 µm or greater, and still more preferably 0.3 µm or greater. In addition, the total film thickness is preferably 200 µm or less, preferably 50 µm or less, preferably 30 µm or less, and preferably 5 µm or less. The total film thickness can be set to a range in which the above-described values are appropriately combined.

In the invention, in a case where the photosensitive layer is provided in a thick film shape (in the photosensitive layer 13B and 13C), the light absorbing agent that is included in the photosensitive layer may function as a hole transporting material.

The amount of the perovskite compound used may be set to an amount capable of covering at least a part of a surface of the first electrode 1, and preferably an amount capable of covering the entirety of the surface.

### [Light Absorbing Agent of Photosensitive Layer]

The photosensitive layer 13 contains at least one kind of perovskite compound (also referred to as "perovskite-type light absorbing agent") that includes "an element of Group 1 in the periodic table or a cationic organic group A", a metal atom M other than the element of Group 1 in the periodic table", and "an anionic atom or atomic group X" as the light absorbing agent.

In the perovskite compound, the element of Group 1 in the periodic table or the cationic organic group A, the metal atom M, and the anionic atom or atomic group X exists as individual constituent ions of a cation (for convenience, may be referred to as "cation A"), a metal cation (for convenience, may be referred to as "cation M"), and an anion (for convenience, may be referred to as "anion X") in the perovskite-type crystal structure.

In the invention, the cationic organic group represents an organic group having a property of becoming a cation in the perovskite-type crystal structure, and the anionic atom or atomic group represents an atom or atomic group that has a property of becoming an anion in the perovskite-type crystal structure.

In the perovskite compound that is used in the invention, the cation A represents a cation of an element of Group 1 in the periodic table or an organic cation that is composed of a cationic organic group A. The cation A is preferably an organic cation.

The cation of an element of Group 1 in the periodic table is not particularly limited, and examples thereof include cations (Li⁺, Na⁺, K⁺, and Cs⁺) of individual elements of lithium (Li), sodium (Na), potassium (K), and cesium (Cs), and the cation (Cs⁺) of cesium is more preferable.

The organic cation is not particularly limited as long as the organic cation is a cation of an organic group having the above-described property, but an organic cation of a cationic organic group represented by the following Formula (A1) is more preferable.

Formula (A1): R^{1a}-NH₃⁺

In Formula (A1), R^{1a} represents a substituent group. R^{1a} is not particularly limited as long as R^{1a} is an organic group, but an alkyl group, a cycloalkyl group, an alkenyl group, an alkynyl group, an aryl group, a heteroaryl group, or a group represented by the following Formula (A2) is preferable. Among these, the alkyl group and a group represented by the following Formula (A2) are more preferable.

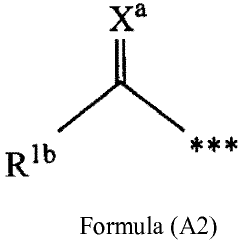

In Formula (A2), X^{a} represents NR^{1c}, an oxygen atom, or a sulfur atom. R^{1b} and R^{1c} each independently represent a hydrogen atom or a substituent group. *** represents bonding with a nitrogen atom in Formula (A1).

In the invention, as the organic cation of the cationic organic group A, an organic ammonium cation (R^{1a}-NH₃⁺) composed of an ammonium cationic organic group A obtained through bonding between R^{1a} and NH₃ in Formula (A1) is preferable. In a case where the organic ammonium cation can employ a resonance structure, the organic cation further includes a cation having the resonance structure in addition to the organic ammonium cation. For example, in a case where X^{a} is NH (R^{1c} is a hydrogen atom) in a group represented by Formula (A2), the organic cation also includes an organic amidinium cation that is one of a resonance structure of the organic ammonium cation in addition to the organic ammonium cation of the ammonium cationic organic group obtained through bonding between the group represented by Formula (A2) and NH₃.

Examples of the organic amidinium cation composed of the amidinium cationic organic group include a cation represented by the following Formula (A^{am}). In this specification, the cation represented by the following Formula (A^{am}) may be noted as "R^{1b}C(=NH)-NH₃" for convenience.

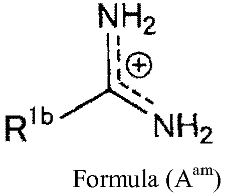

The alkyl group is preferably an alkyl group having 1 to 30 carbon atoms, more preferably an alkyl group having 2 to 18 carbon atoms, and still more preferably an alkyl group having 4 to 18 carbon atoms. Examples of the alkyl group include methyl, ethyl, propyl, isopropyl, butyl, tert-butyl, pentyl, hexyl, decyl, and the like.

The cycloalkyl group is preferably a cycloalkyl group having 3 to 8 carbon atoms, and examples thereof include cyclopropyl, cyclopentyl, cyclohexyl, and the like.

The alkenyl group is preferably an alkenyl group having 2 to 18 carbon atoms, and more preferably an alkenyl group having 2 to 6 carbon atoms. Examples of the alkenyl group include vinyl, allyl, butenyl, hexenyl, and the like.

The alkynyl group is preferably an alkynyl group having 2 to 18 carbon atoms, and more preferably an alkynyl group having 2 to 4 carbon atoms. Examples of the alkynyl group include ethynyl, butynyl, hexynyl, and the like.

The aryl group is preferably an aryl group having 6 to 14 carbon atoms, and more preferably an aryl group having 6 to 12 carbon atoms, and examples thereof include phenyl.

The heteroaryl group includes a group composed of an aromatic hetero ring alone, and a group composed of a condensed hetero ring obtained through condensing of another ring, for example, an aromatic ring, an aliphatic ring, or a hetero ring with the aromatic hetero ring.

As the ring-constituting hetero atom that constitutes the aromatic hetero ring, a nitrogen atom, an oxygen atom, or a sulfur atom is preferable. In addition, with regard to the number of ring members of the aromatic hetero ring, three-membered to eight-membered rings are preferable, and a five-membered ring or a six-membered ring is more preferable.

Examples of the five-membered aromatic hetero ring and the condensed hetero ring including the five-membered aromatic hetero ring include respective cyclic groups of a pyrrole ring, an imidazole ring, a pyrazole ring, an oxazole ring, a thiazole ring, a triazole ring, a furan ring, a thiophene ring, a benzimidazole ring, a benzoxazole ring, a benzothiazole ring, an indoline ring, and an indazole ring. In addition, examples of the six-membered aromatic hetero ring and the condensed hetero ring including the six-membered aromatic hetero ring include respective cyclic groups of a pyridine ring, a pyrimidine ring, a pyrazine ring, a triazine ring, a quinoline ring, and a quinazoline ring.

In the group represented by Formula (A2), X^{a} represents NR^{1c}, an oxygen atom, or a sulfur atom, and NR^{1c} is preferable as X^{a}. Here, R^{1c} represents a hydrogen atom or a substituent group. R^{1c} is preferably a hydrogen atom, an alkyl group, a cycloalkyl group, an alkenyl group, an alkynyl group, an aryl group, or a heteroaryl group, and more preferably a hydrogen atom.

R^{1b} represents a hydrogen atom or a substituent group, and is preferably a hydrogen atom. Examples of the substituent group that can be employed as R^{1b} include an amino group, an alkyl group, a cycloalkyl group, an alkenyl group, an alkynyl group, an aryl group, a heteroaryl group.

An alkyl group, a cycloalkyl group, an alkenyl group, an alkynyl group, an aryl group, and a heteroaryl group that can be respectively employed by R^{1b} and R^{1c} are the same as the respective groups of R^{1a}, and preferred examples thereof are the same as described above.

Examples of the group represented by Formula (A2) include a (thio)acyl group, a (thio)carbamoyl group, an imidoyl group, and an amidino group.

Examples of the (thio)acyl group include an acyl group and a thioacyl group. The acyl group is preferably an acyl group having a total of 1 to 7 carbon atoms, and examples thereof include formyl, acetyl, propionyl, hexanoyl, and the like. The thioacyl group is preferably a thioacyl group having a total of 1 to 7 carbon atoms, and examples thereof include thioformyl, thioacetyl, thiopropionyl, and the like.

Examples of the (thio)carbamoyl group include a carbamoyl group and a thiocarbamoyl group (H₂NC(=S)-).

The imidoyl group is a group represented by R^{1b}-C(=NR^{1c})-, and it is preferable that R^{1b} and R^{1c} are respectively a hydrogen atom and an alkyl group. More preferably, the alkyl group is the same as the alkyl group as R^{1a}. Examples thereof include formimidoyl, acetoimidoyl, propionimidoyl (CH₃CH₂C(=NH)-), and the like. Among these, formimidoyl is preferable.

The amidino group as the group represented by Formula (2) has a structure in which R^{1b} of the imidoyl group is an amino group and R^{1c} is a hydrogen atom.

The entirety of the alkyl group, the cycloalkyl group, the alkenyl group, the alkynyl group, the aryl group, the heteroaryl group, and the group represented by Formula (A2), which can be employed as R^{1a}, may have a substituent group. The substituent group W^{P}, which R^{1a} may have, is not particularly limited, and examples thereof include an alkyl group, a cycloalkyl group, an alkenyl group, an alkynyl group, an aryl group, a heterocyclic group, an alkoxy group, an alkylthio group, an amino group, an alkylamino group, an arylamino group, an acyl group, an alkylcarbonyloxy group, an aryloxy group, an alkoxycarbonyl group, an aryloxycarbonyl group, an acylamino group, a sulfonamido group, a carbamoyl group, a sulfamoyl group, a halogen atom, a cyano group, a hydroxy group, a mercapto group, and a carboxy group. The substituent group, which R^{1a} may have, may be additionally substituted with a substituent group.

In the perovskite compound that is used in the invention, the metal cation M is not particularly limited as long as the metal cation M is a cation of a metal atom other than elements of Group 1 in the periodic table and is a cation of a metal atom that can employ the perovskite-type crystal structure. Examples of the metal atom include metal atoms such as calcium (Ca), strontium (Sr), cadmium (Cd), copper (Cu), nickel (Ni), manganese (Mn), iron (Fe), cobalt (Co), palladium (Pd), germanium (Ge), tin (Sn), lead (Pb), ytterbium (Yb), europium (Eu), indium (In), titanium (Ti), bismuth (Bi), and thallium (Tl). Among these, as the metal atom M, a Pb atom or a Sn atom is more preferable. M may be one kind of metal atom, or two or more kinds of metal atoms. In a case where M includes two or more kinds of metal atoms, two kinds including the Pb atom and the Sn atom are preferable. A ratio of the metal atoms at this time is not particularly limited.

In the perovskite compound that is used in the invention, the anion X represents an anion of an anionic atom or atomic group X. Preferred examples of the anion include anions of halogen atoms, and anions of individual atomic groups of NCS⁻, NCO⁻, CH₃COO⁻, and HCOO⁻. Among these, the anions of halogen atoms are more preferable. Examples of the halogen atoms include a fluorine atom, a chlorine atom, a bromine atom, an iodine atom, and the like.

The anion X may be an anion of one kind of anionic atom or atomic group, or anions of two or more kinds of anionic atoms or atomic groups. In a case where the anion X is an anion of one kind of anionic atom or atomic group, an anion of an iodine atom is preferable. On the other hand, in a case where the anion X includes anions of two or more kinds of anionic atoms or atomic groups, anions of two kinds of halogen atoms, particularly, an anion of a chlorine atom and an anion of an iodine atom are preferable. A ratio between two or more kinds of anions is not particularly limited.

As the perovskite compound that is used in the invention, a perovskite compound, which has a perovskite-type crystal structure including the above-described constituent ions and is represented by the following Formula (I), is preferable.

Formula (I): AₐMₘXₓ

In Formula (I), A represents an element of Group 1 in the periodic table or a cationic organic group. M represents a metal atom other than the element of Group 1 in the periodic table. X represents an anionic atom or atomic group.
a represents 1 or 2, m represents 1, and a, m, and x satisfy a relationship of a+2m=x.

In Formula (I), the element of Group 1 in the periodic table or the cationic organic group A forms a cation A of the perovskite-type crystal structure. Accordingly, there is no particular limitation as long as the element of Group 1 in the periodic table and the cationic organic group A are elements or groups which become the cation A and can constitute the perovskite-type crystal structure. The element of Group 1 in the periodic table or the cationic organic group A is the same as the element of Group 1 in the periodic table or the cationic organic group which is described in the above-described cation A, and preferred examples thereof are the same as described above.

The metal atom M is a metal atom that forms the metal cation M of the perovskite-type crystal structure. Accordingly, the metal atom M is not particularly limited as long as the metal atom M is an atom other than the element of Group 1 in the periodic table, becomes the metal cation M, and constitutes the perovskite-type crystal structure. The metal atom M is the same as the metal atom that is described in the above-described metal cation M, and preferred examples thereof are the same as described above.

The anionic atom or atomic group X forms the anion X of the perovskite-type crystal structure. Accordingly, the anionic atom or atomic group X is not particularly limited as long as the anionic atom or atomic group X is an atom or atomic group that becomes the anion X and can constitute the perovskite-type crystal structure. The anionic atom or atomic group X is the same as the anionic atom or atomic group which is described in the anion X, and preferred examples thereof are the same as described above.

The perovskite compound represented by Formula (I) is a perovskite compound represented by the following Formula (1-1) in a case where a is 1, or a perovskite compound represented by the following Formula (1-2) in a case where a is 2.

Formula (I-1): AMX₃

Formula (1-2): A₂MX₄

In Formula (I-1) and Formula (1-2), A represents an element of Group 1 in the periodic table or a cationic organic group. A is the same as A in Formula (I), and preferred examples thereof are the same as described above.

M represents a metal atom other than the element of Group 1 in the periodic table. M is the same as M in Formula (I), and preferred examples thereof are the same as described above.

X represents an anionic atom or atomic group. X is the same as X in Formula (I), and preferred examples thereof are the same as described above.

The perovskite compound that is used in the invention may be any one of the compound represented by Formula (1-1) and the compound represented by Formula (1-2), or a mixture thereof. Accordingly, in the invention, at least one kind of the perovskite compound may exist as the light absorbing agent, and there is no need for clear and strict distinction on that the perovskite compound is which compound by using a composition formula, a molecular formula, a crystal structure, and the like.

Hereinafter, specific examples of the perovskite compound that can be used in the invention will be exemplified, but the invention is not limited to the specific examples. In the following description, the perovskite compound is classified into the compound represented by Formula (I-1) and the compound represented by Formula (1-2). However, even the compound exemplified as the compound represented by Formula (I-1) may be the compound represented by Formula (1-2) in accordance with synthesis conditions, or may be a mixture of the compound represented by Formula (I-1) and the compound represented by Formula (1-2). Similarly, even the compound exemplified as the compound represented by Formula (1-2) may be the compound represented by Formula (1-1), or may be a mixture of the compound represented by Formula (I-1) and the compound represented by Formula (1-2).

Specific examples of the compound represented by Formula (I-1) include CH₃NH₃PbCl₃, CH₃NH₃PbBr₃, CH₃NH₃PbI₃, CH₃NH₃PbBrI₂, CH₃NH₃PbBr₂I, CH₃NH₃SnBr₃, CH₃NH₃SnI₃, and CH(=NH)NH₃PbI₃.

Specific examples of the compound represented by Formula (1-2) include (C₂H₅NH₃)₂PbI₄, (CH₂=CHNH₃)₂PbI₄, (CH=CNH₃)₂PbI₄, (n-C₃H₇NH₃)₂PbI₄, (n-C₄H₉NH₃)₂PbI₄, (C₁₀H₂₁NH₃)₂PbI₄, (C₆H₅NH₃)₂PbI₄, (C₆H₅CH₂CH₂NH₃)₂PbI₄, (C₆H₃F₂NH₃)₂PbI₄, (C₆F₅NH₃)₂PbI₄, and (C₄H₃SNH₃)₂PbI₄. Here, C₄H₃SNH₃ in (C₄H₃SNH₃)₂PbI₄ represents aminothiophene.

The perovskite compound can be synthesized from a compound represented by Formula (II) and a compound represented by Formula (III).

Formula (II): AX

Formula (III): MX₂

In Formula (II), A represents an element of Group 1 in the periodic table, or a cationic organic group. A is the same as A in Formula (I), and preferred examples thereof are the same as described above. In Formula (II), X represents an anionic atom or atomic group. X is the same as X in Formula (I), and preferred examples thereof are the same as described above.

In Formula (III), M represents a metal atom other than the element of Group 1 in the periodic table. M is the same as M in Formula (I), and preferred examples thereof are the same as described above. In Formula (III), X represents an anionic atom or atomic group. X is the same as X in Formula (I), and preferred examples thereof are the same as described above.

With regard to a method of synthesizing the perovskite compound, for example, Non-Patent Literature 1 can be exemplified. In addition, Akihiro Kojima, Kenjiro Teshima, Yasuo Shirai, and Tsutomu Miyasaka, "Organometal Halide Perovskites as Visible-Light Sensitizers for Photovoltaic Cells", J. Am. Chem. Soc., 2009, 131(17), p. 6050 to 6051 can be exemplified.

The amount of the light absorbing agent used is preferably set to an amount capable of covering at least a part of the surface of the first electrode 1, and more preferably an amount capable of covering the entirety of the surface.

The amount of the perovskite compound contained in the photosensitive layer 13 is typically 1% to 100% by mass.

### [Hole Transport Layer]

As in the photoelectric conversion elements 10A to 10D, the photoelectric conversion element of the invention includes the hole transport layer 3 between the first electrode 1 and the second electrode 2. The hole transport layer 3 is preferably provided between the photosensitive layer 13 of the first electrode 1 and the second electrode 2.

The hole transport layer 3 includes a function of supplementing electrons to an oxidized substance of the light absorbing agent, and is preferably a solid-shaped layer (solid hole transport layer).

As described above, the hole transporting material, which is used in the hole transport layer of the invention, includes a compound including a condensed polycyclic aromatic group having the number of rings of 4 or greater, and at least two rings in the condensed polycyclic aromatic group are hetero rings including at least one atom selected from the group consisting of a sulfur atom, a nitrogen atom, a selenium atom, and an oxygen atom. The condensed polycyclic aromatic group includes at least one structure selected from the group consisting of a benzene ring, a naphthalene ring, an anthracene ring, and a phenanthrene ring as a partial structure.

The hole transporting material having the above-described specific structure has high hydrophobicity, and thus contributes to an improvement of moisture resistance of the photoelectric conversion element.

In the compound that includes the condensed polycyclic aromatic group having the number of rings of 4 or greater, and constitutes the hole transporting material of the invention, at least two rings in the condensed polycyclic aromatic group are hetero rings including at least one atom selected from the group consisting of a sulfur atom, a nitrogen atom, a selenium atom, and an oxygen atom, and the other rings are carbon rings.

With regard to the number of ring members of the hetero rings including at least one atom which is selected from the group consisting of a sulfur atom, a nitrogen atom, a selenium atom, and an oxygen atom, and constitutes at least two rings in the condensed polycyclic aromatic group, three-membered to eight-membered rings are preferable, and a five-membered ring or a six-membered ring is more preferable.

Examples of the five-membered hetero ring include respective cyclic groups of a pyrrole ring, an imidazole ring, a pyrazole ring, an oxazole ring, a thiazole ring, a triazole ring, a furan ring, a thiophene ring, and a selenophene ring. Examples of the six-membered hetero ring include respective cyclic groups of a pyridine ring, a pyrimidine ring, a pyrazine ring, a triazine ring, a thiazine ring, an oxazine ring, and a selenazine ring.

As a partial structure which the hole transporting material can include, at least one structure selected from the group consisting of the benzene ring, the naphthalene ring, and the phenanthrene ring is more preferable than the anthracene ring, and it is preferable that the anthracene ring is not included.

The number of rings of the condensed polycyclic aromatic group included in the hole transporting material is preferably 5 or 6. In addition, it is preferable that at least two hetero rings, which constitute the condensed polycyclic aromatic group included in the hole transporting material, respectively include one hetero atom, and more preferably a sulfur atom.

The hole transporting material having the structure advantageously operates to improve moisture resistance of the photoelectric conversion element.

The hole transporting material includes at least one kind of compound represented by any one of Formula 1 to Formula 16 as described above.

A substituent group, which the compounds represented by Formula 1 to Formula 16 may include, is not particularly limited as long as the substituent group is an organic group, and an alkyl group, a cycloalkyl group, an alkenyl group, an alkynyl group, an alkoxy group, an aryloxy group, an alkylthio group, an arylthio group, an amino group, an acyl group, an acyloxy group, an alkoxycarbonyl group, an aryloxycarbonyl group, a carbamoyl group, an acylamino group, a sulfonyl group, an aryl group, a heteroaryl group, or a silyl group is preferable.

The alkyl group is preferably an alkyl group having 1 to 30 carbon atoms, more preferably an alkyl group having 2 to 18 carbon atoms, and still more preferably an alkyl group having 4 to 18 carbon atoms. Examples of the alkyl group include methyl, ethyl, propyl, isopropyl, butyl, tert-butyl, pentyl, hexyl, decyl, and the like. As the alkyl group, linear alkyl having 8 or more carbon atoms or branched alkyl having 4 or more carbon atoms is more preferable. As the linear alkyl having 8 or more carbon atoms, linear alkyl having 8 to 30 carbon atoms is preferable, and linear alkyl having 8 to 18 carbon atoms is more preferable. Examples of the linear alkyl include n-octyl, n-decyl, n-undecyl, n-octadecyl, and the like. As the branched alkyl having 4 or more carbon atoms, branched alkyl having 4 to 30 is preferable, and branched alkyl having 4 to 12 carbon atoms is more preferable. Examples of the branched alkyl include tert-butyl, 2-ethylhexyl, 3,7-dimethyloctyl, neopentyl, 2-octyldodecyl, and the like.

The cycloalkyl group is preferably a cycloalkyl group having 3 to 8 carbon atoms, and examples thereof include cyclopropyl, cyclopentyl, cyclohexyl, and the like.

The alkenyl group is preferably an alkenyl group having 2 to 18 carbon atoms, and more preferably an alkenyl group having 2 to 6 carbon atoms. Examples of the alkenyl group include vinyl, allyl, butenyl, pentenyl, hexenyl, and the like.

The alkynyl group is preferably an alkynyl group having 2 to 18 carbon atoms, and more preferably an alkynyl group having 2 to 4 carbon atoms. Examples of the alkynyl group include ethynyl, butynyl, pentynyl, hexynyl, and the like.

The alkoxy group is preferably an alkoxy group having 1 to 30 carbon atoms, and more preferably an alkoxy group having 4 to 18 carbon atoms. Examples of the alkoxy group include methoxy, ethoxy, isopropyloxy, tert-butyloxy, pentyloxy, hexyloxy, heptyloxy, octyloxy, benzyloxy, and the like.

The aryloxy group is preferably an aryloxy group having 6 to 14 carbon atoms, more preferably an aryloxy group having 6 to 12 carbon atoms, and examples thereof include phenoxy.

The alkylthio group is preferably a alkylthio group having 1 to 30 carbon atoms, and more preferably an alkylthio group having 4 to 18 carbon atoms. Examples of the alkylthio group include methylthio, ethylthio, isopropylthio, t-butylthio, octylthio, and the like.

The arylthio group is preferably an arylthio group having 6 to 14 carbon atoms, more preferably an arylthio group having 6 to 12 carbon atoms, and examples thereof include phenylthio.

The amino group is preferably an amino group having 0 to 20 carbon atoms, and includes an alkylamino group, an alkenylamino group, an alkynylamino group, a cycloalkylamino group, a cycloalkenylamino group, an arylamino group, and a heterocyclic amino group. Examples of the amino group include amino, N,N-dimethylamino, N,N-diethylamino, N-ethylamino, N-allylamino, N-(2-propynyl)amino, N-cyclohexylamino, N-cyclohexenylamino, anilino, pyridylamino, imidazolylamino, benzimidazolylamino, thiazolylamino, benzothiazolylamino, and triazinylamino.

The acyl group is preferably an acyl group having 1 to 20 carbon atoms, and examples thereof include acetyl, cyclohexylcarbonyl, and benzoyl.

The acyloxy group is preferably an acyloxy group having 1 to 20 carbon atoms, and examples thereof include acetyloxy, cyclohexylcarbonyloxy, and benzoyloxy.

The alkoxycarbonyl group is preferably an alkoxycarbonyl group having 2 to 20 carbon atoms, and examples thereof include ethoxycarbonyl and 2-ethylhexyloxycarbonyl.

The aryloxycarbonyl group is preferably an aryloxycarbonyl group having 6 to 20 carbon atoms, and examples thereof include phenyloxycarbonyl and naphthyloxycarbonyl.

The carbamoyl group is preferably a carbamoyl group of alkyl, cycloalkyl, or aryl which has 1 to 20 carbon atoms, and examples thereof include N,N-dimethylcarbamoyl, N-cyclohexylcarbamoyl, and N-phenylcarbamoyl.

The acylamino group is preferably an acylamino group having 1 to 20 carbon atoms, and examples thereof include acetylamino, cyclohexylcarbonylamino, and benzoylamino.

The sulfonyl group includes alkyl or arylsulfonyl group and preferably has 1 to 20 carbon atoms. Examples of the sulfonyl group include methylsulfonyl, ethylsulfonyl, cyclohexylsulfonyl, and benzenesulfonyl.

The silyl group is preferably a silyl group that has 1 to 20 carbon atoms and is substituted with alkyl, aryl, alkoxy, and aryloxy. Examples of the silyl group include trimethylsilyl, triethylsilyl, triisopropylsilyl, triphenylsilyl, diethylbenzylsilyl, and dimethylphenylsilyl.

The aryl group is preferably an aryl group having 6 to 14 carbon atoms, more preferably an aryl group having 6 to 12 carbon atoms, and examples thereof include phenyl and naphthyl.

The heteroaryl group includes a group composed of an aromatic hetero ring alone, and a group composed of a condensed hetero ring obtained through condensing of another ring, for example, an aromatic ring, an aliphatic ring, or a hetero ring with the aromatic hetero ring.

As the ring-constituting hetero atom that constitutes the aromatic hetero ring, a nitrogen atom, an oxygen atom, or a sulfur atom is preferable. In addition, with regard to the number of ring members of the aromatic hetero ring, three-membered to eight-membered rings are preferable, and a five-membered ring or a six-membered ring is more preferable.

Examples of the five-membered aromatic hetero ring and the condensed hetero ring including the five-membered aromatic hetero ring include respective cyclic groups of a pyrrole ring, an imidazole ring, a pyrazole ring, an oxazole ring, a thiazole ring, a triazole ring, a furan ring, a thiophene ring, a selenophene ring, a benzimidazole ring, a benzoxazole ring, a benzothiazole ring, an indoline ring, and an indazole ring. In addition, examples of the six-membered aromatic hetero ring and the condensed hetero ring including the six-membered aromatic hetero ring include respective cyclic groups of a pyridine ring, a pyrimidine ring, a pyrazine ring, a triazine ring, a quinoline ring, and a quinazoline ring.

The compounds represented by Formula 1 to Formula 16 as the hole transporting material include at least one group represented by Formula (W): -L^{W}-R^{W} described above as a substituent group. As R^{W} that constitutes Formula (W), a hydrogen atom, an alkyl group, an aryl group, and a heteroaryl group are preferable, and the alkyl group is more preferable. Specific examples and preferred ranges of the respective groups are the same as in the groups described in the substituent groups which the compound represented by Formula 1 to Formula 16 may have.

The alkyl group is preferably an alkyl group having 1 to 30 carbon atoms, more preferably an alkyl group having 2 to 18 carbon atoms, and still more preferably an alkyl group having 4 to 18 carbon atoms. Examples thereof include methyl, ethyl, propyl, isopropyl, butyl, tert-butyl, pentyl, hexyl, decyl, and the like. As the alkyl group, linear alkyl having 8 or more carbon atoms or branched alkyl having 4 or more carbon atoms is more preferable. As the linear alkyl having 8 or more carbon atoms, linear alkyl having 8 to 30 carbon atoms is preferable, and linear alkyl having 8 to 18 carbon atoms is more preferable. Examples of the linear alkyl include n-octyl, n-decyl, n-undecyl, n-octadecyl, and the like. As the branched alkyl having 4 or more carbon atoms, branched alkyl having 4 to 30 carbon atoms is preferable, and branched alkyl having 4 to 12 carbon atoms is more preferable. Examples of the branched alkyl include tert-butyl, 2-ethylhexyl, 3,7-dimethyloctyl, neopentyl, 2-octyldodecyl, and the like.

A substituent group as R' or R^{N}, which is employed in a divalent linking group represented by any one of Formula L-1 to L-25 or a divalent linking group in which two or more divalent linking groups each being represented by any one of Formula L-1 to L-25 are bonded to each other as L^{W} that constitutes the group represented by Formula (W): -L^{W}-R^{W}, is not particularly limited as long as the substituent group is an organic group, and an alkyl group, a cycloalkyl group, an alkenyl group, an alkynyl group, an aryl group, or a heteroaryl group is preferable.

The alkyl group is preferably an alkyl group having 1 to 30 carbon atoms, more preferably an alkyl group having 2 to 18 carbon atoms, and still more preferably an alkyl group having 4 to 18 carbon atoms. Examples of the alkyl group include methyl, ethyl, propyl, isopropyl, butyl, tert-butyl, pentyl, hexyl, decyl, and the like. As the alkyl group, linear alkyl having 8 or more carbon atoms or branched alkyl having 4 or more carbon atoms is more preferable. As the linear alkyl having 8 or more carbon atoms, linear alkyl having 8 to 30 carbon atoms is preferable, and linear alkyl having 8 to 18 carbon atoms is more preferable. Examples of the linear alkyl include n-octyl, n-decyl, n-undecyl, n-octadecyl, and the like. As the branched alkyl having 4 or more carbon atoms, branched alkyl having 4 to 30 carbon atoms is preferable, and branched alkyl having 4 to 12 carbon atoms is more preferable. Examples of the branched alkyl include tert-butyl, 2-ethylhexyl, 3,7-dimethyloctyl, neopentyl, 2-octyldodecyl, and the like.

The cycloalkyl group is preferably a cycloalkyl group having 3 to 8 carbon atoms, and examples thereof include cyclopropyl, cyclopentyl, cyclohexyl, and the like.

The alkenyl group is preferably an alkenyl group having 2 to 18 carbon atoms, and more preferably an alkenyl group having 2 to 6 carbon atoms. Examples of the alkenyl group include vinyl, allyl, butenyl, pentenyl, hexenyl, and the like.

The alkynyl group is preferably an alkynyl group having 2 to 18 carbon atoms, and more preferably an alkynyl group having 2 to 4 carbon atoms. Examples of the alkynyl group include ethynyl, butynyl, pentynyl, hexynyl, and the like.

The aryl group is preferably an aryl group having 6 to 14 carbon atoms, more preferably an aryl group having 6 to 12 carbon atoms, and examples thereof include phenyl and naphthyl.

The heteroaryl group includes a group composed of an aromatic hetero ring alone, and a group composed of a condensed hetero ring obtained through condensing of another ring, for example, an aromatic ring, an aliphatic ring, or a hetero ring with the aromatic hetero ring.

As the ring-constituting hetero atom that constitutes the aromatic hetero ring, a nitrogen atom, an oxygen atom, or a sulfur atom is preferable. In addition, with regard to the number of ring members of the aromatic hetero ring, three-membered to eight-membered rings are preferable, and a five-membered ring or a six-membered ring is more preferable.

Examples of the five-membered aromatic hetero ring and the condensed hetero ring including the five-membered aromatic hetero ring include respective cyclic groups of a pyrrole ring, an imidazole ring, a pyrazole ring, an oxazole ring, a thiazole ring, a triazole ring, a furan ring, a thiophene ring, a selenophene ring, a benzimidazole ring, a benzoxazole ring, a benzothiazole ring, an indoline ring, and an indazole ring. In addition, examples of the six-membered aromatic hetero ring and the condensed hetero ring including the six-membered aromatic hetero ring include respective cyclic groups of a pyridine ring, a pyrimidine ring, a pyrazine ring, a triazine ring, a quinoline ring, and a quinazoline ring.

R^{Si} in Formula (L-25) represents a hydrogen atom, an alkyl group, an alkenyl group, or an alkynyl group, and specific examples and preferred ranges of the respective groups are the same as in the groups described in R' or R^{N}.

It is preferable that the hole transporting material includes at least one substituent group represented by Formula L-2', L-3', L-4', or L-6' described above, and preferably a substituent group represented by Formula L-2' or L-6'. Rw1 to Rw4 in Formula L-2', L-3', L-4', or L-6' represent a hydrogen atom, an alkyl group, an aryl group, and a heteroaryl group. Specific examples and preferred ranges of the respective groups are the same as in the groups described in Rw.

The hole transporting material that includes the substituent groups contributes to an improvement of moisture resistance in the photoelectric conversion element.

It is preferable that the hole transporting material includes a linear alkyl portion having 8 or more carbon atoms, or a branched alkyl portion having 4 or more carbon atoms. As the linear alkyl having 8 or more carbon atoms, linear alkyl having 8 to 30 carbon atoms is preferable, and linear alkyl having 8 to 18 carbon atoms is more preferable. Examples of the linear alkyl include n-octyl, n-decyl, n-undecyl, n-octadecyl, and the like. As the branched alkyl having 4 or more carbon atoms, branched alkyl having 4 to 30 carbon atoms is preferable, and branched alkyl having 4 to 12 carbon atoms is more preferable. Examples of the branched alkyl include tert-butyl, 2-ethylhexyl, 3,7-dimethyloctyl, neopentyl, 2-octyldodecyl, and the like.

The hole transporting material that includes the linear alkyl portion having 8 or more carbon atoms, or the branched alkyl portion having 4 or more carbon atoms enhances hydrophobicity, and thus the hole transporting material contributes an improvement of moisture resistance in the photoelectric conversion element.

A method of synthesizing the hole transporting material of the invention is not particularly limited, and the hole transporting material can be synthesized with reference to a method that is known in the related art. Examples of the method of synthesizing the compounds represented by Formula 1 to Formula 16 as described above include Journal of American Chemical Society, 116, 925(1994), Journal of Chemical Society, 221(1951), Org. Lett., 2001, 3, 3471, Macromolecules, 2010, 43, 6264, Tetrahedron, 2002, 58, 10197, JP2012-513459A, JP2011-46687A, Journal of Chemical Research, miniprint, 3, 601-635(1991), Bull. Chem. Soc. Japan, 64, 3682-3686(1991), Tetrahedron Letters, 45, 2801-2803(2004), EP2251342A, EP2301926A, EP2301921A, KR10-2012-0120886A, J. Org. Chem., 2011, 696, Org. Lett., 2001, 3, 3471, Macromolecules, 2010, 43, 6264, J. Org. Chem., 2013, 78, 7741, Chem. Eur. J., 2013, 19, 3721, Bull. Chem. Soc. Jpn., 1987, 60, 4187, J. Am. Chem. Soc., 2011, 133, 5024, Chem. Eur. J. 2013, 19, 3721, Macromolecules, 2010, 43, 6264-6267, J. Am. Chem. Soc., 2012, 134, 16548-16550, and the like.

Specific examples of the hole transporting material include the following Chemical Formula 1 to Chemical Formula 57.

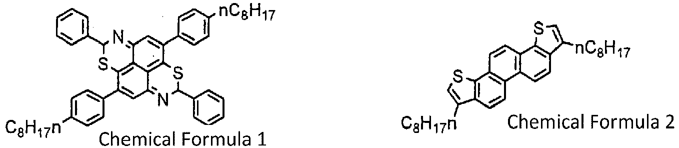

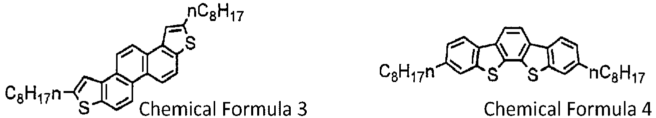

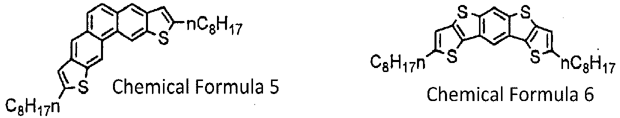

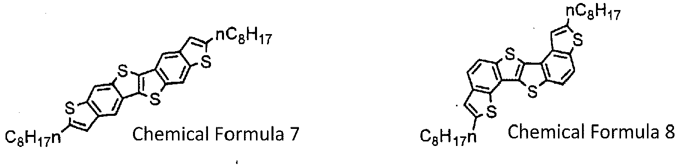

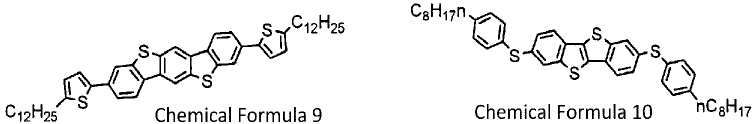

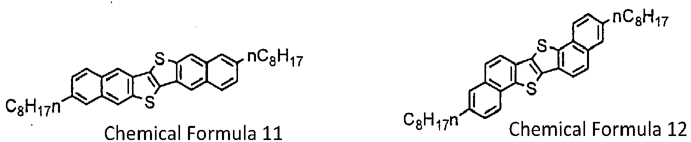

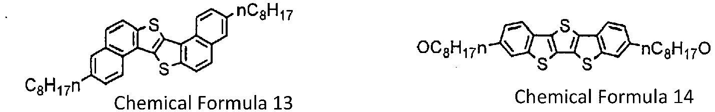

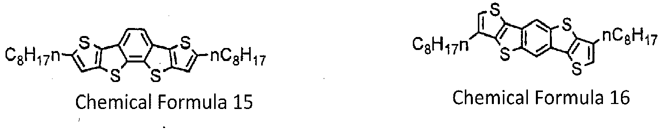

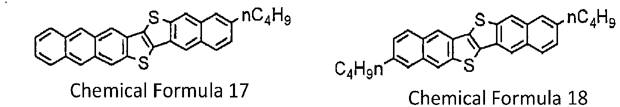

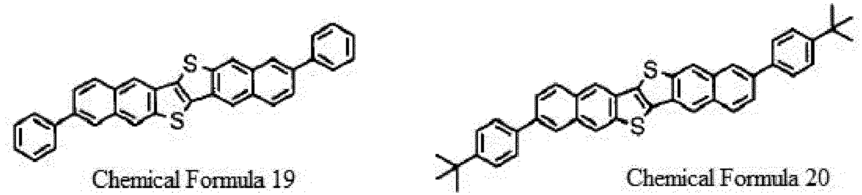

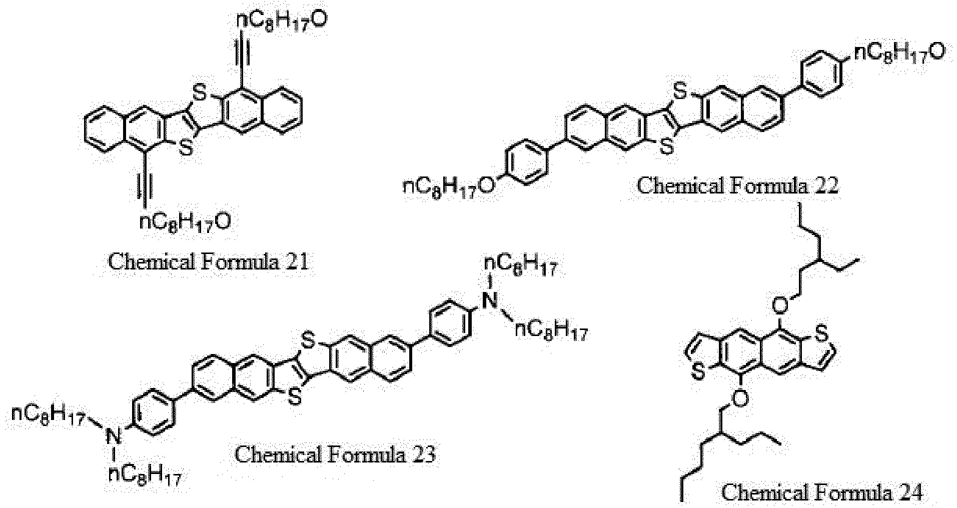

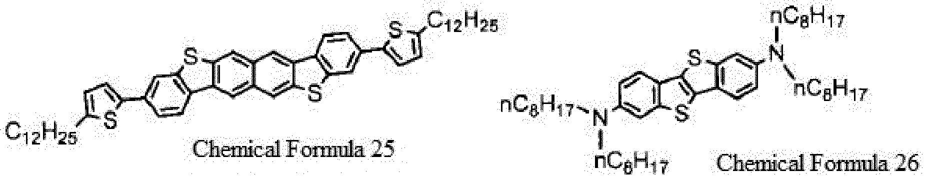

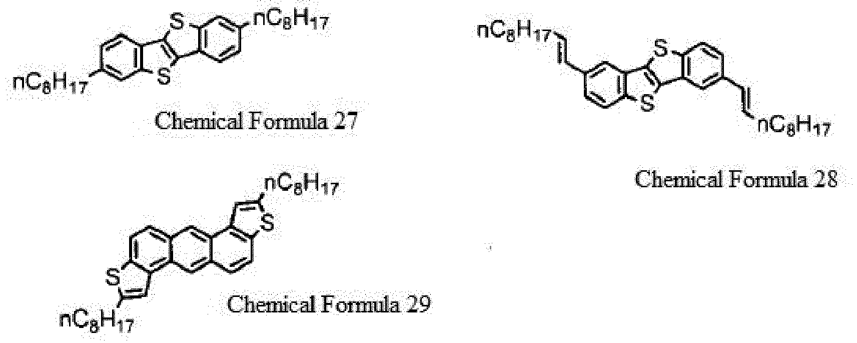

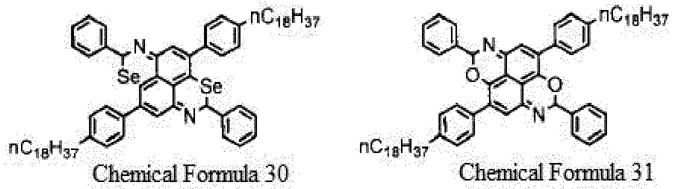

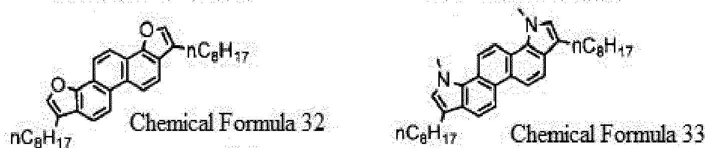

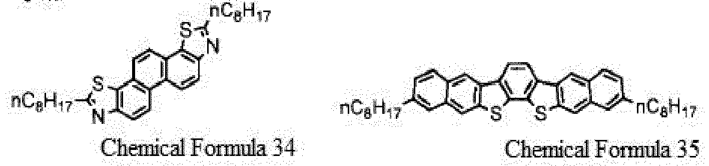

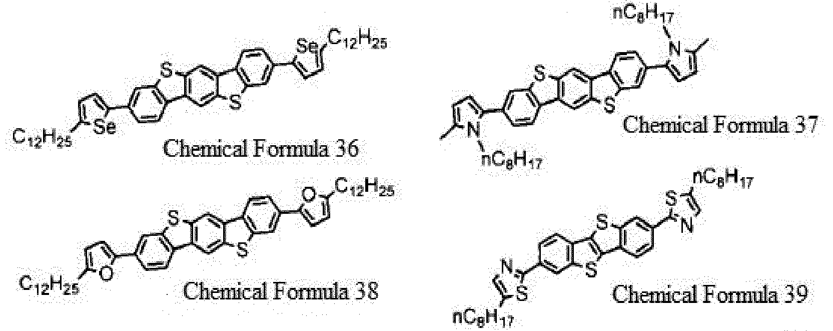

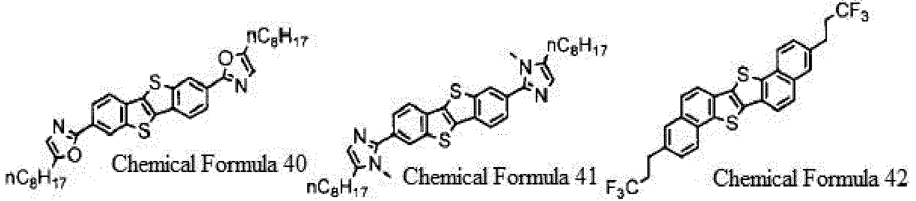

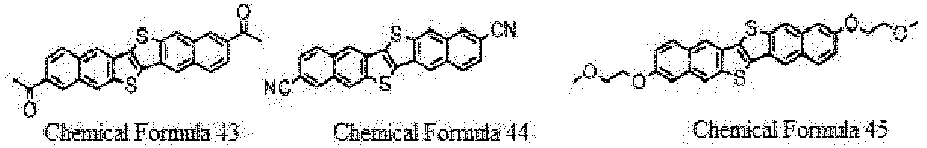

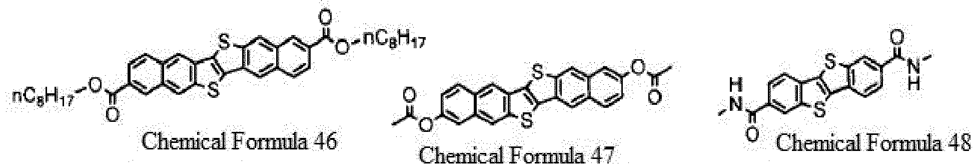

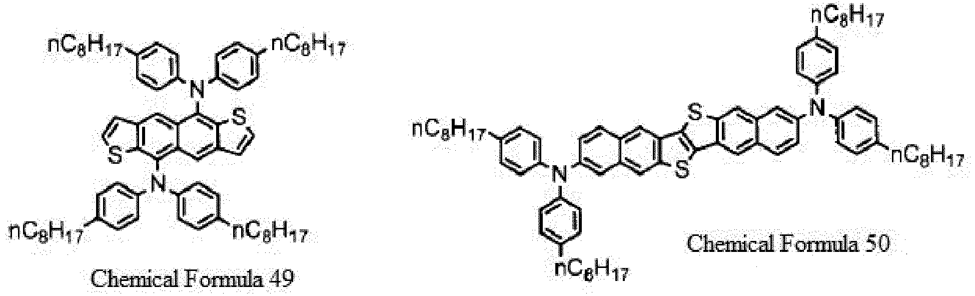

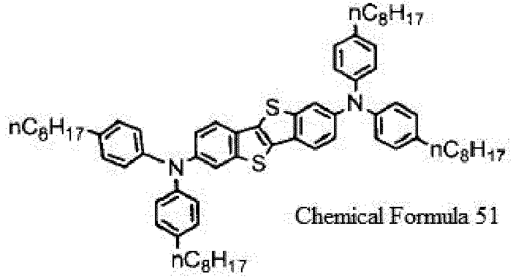

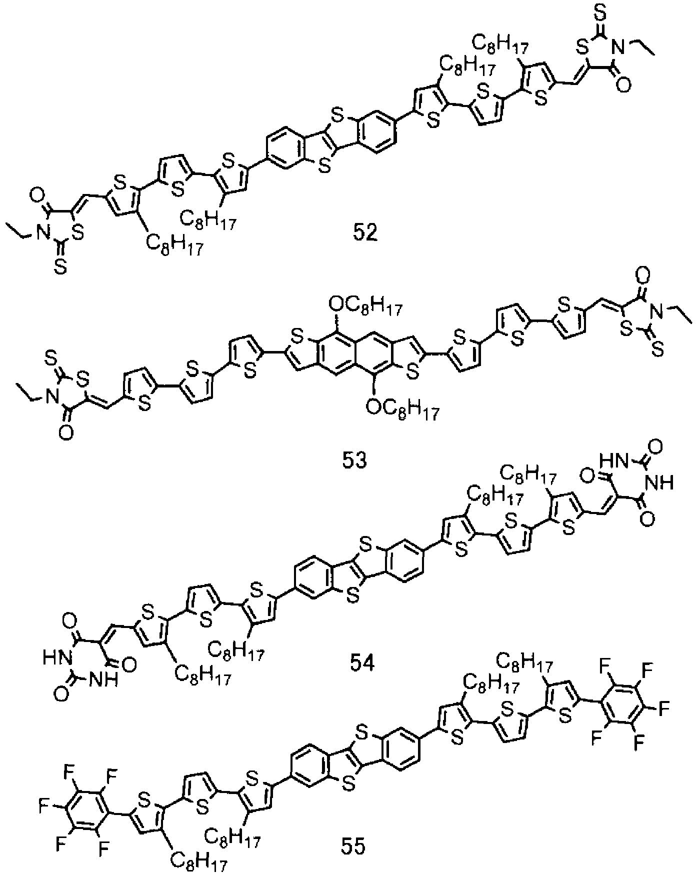

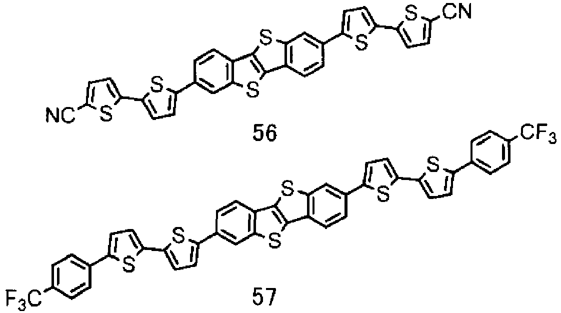

Compounds of Chemical Formula 1 to Chemical Formula 16 respectively correspond to the hole transporting materials represented by Formula 1 to Formula 16. Compounds of Chemical Formula 18 to Chemical Formula 23 and Chemical Formula 50 respectively correspond to the hole transporting material represented by Formula 11. Compounds of Chemical Formula 26-28, 51, 52 and 54-57 correspond to Formula 10. Compounds 30 and 31 are according to Chemical Formula 1 and compounds 33 and 34 are according to Chemical Formula 2. The other compounds are hole transporting materials which are not included in Formula 1 to Formula 16.

Compounds of Chemical Formula 14, Chemical 21, Chemical Formula 22, Chemical Formula 23, Chemical Formula 26, and Chemical Formula 28 respectively include substituent groups of Formulae L-4', L-3', L-4', L-6', L-6', and L-2'.

Compounds other than Chemical Formula 17 to Chemical Formula 19, Chemical Formula 42 to Chemical Formula 45, Chemical Formula 47, and Chemical Formula 48 include an alkyl portion having 8 or more carbon atoms or a branched alkyl portion having 4 or more carbon atoms.

The hole transport layer of the invention may include another hole transporting material. Examples of the other hole transporting material include inorganic materials such as CuI and CuNCS, organic hole transporting materials described in Paragraphs 0209 to 0212 of JP2001-291534A, and the like. Examples of the organic hole transporting material include conductive polymers such as polythiophene, polyaniline, polypyrrole, and polysilane, spiro compounds in which two rings share a central atom such as C or Si having a tetrahedral structure, aromatic amine compounds such as triarylamine, triphenylene compounds, nitrogen-containing heterocyclic compounds, and liquid-crystalline cyano compounds. Among the organic hole transporting materials, an organic hole transporting material which can be applied in a solution state and then has a solid shape is preferable, and specific examples thereof include 2,2',7,7'-tetrakis-(N,N-di-p-methoxyphenylamino)-9,9'-spirobifluorene [2,2',7,7'-tetrakis-(N,N-di-p-methoxyphenylamine)-9,9'-spirobifluorene: spiro-OMeTAD], poly(3-hexylthiophene-2,5-diyl), 4-(diethylamino)benzaldehyde diphenylhydrazone, poly(3,4-ethylenedioxythiophene) (PEDOT), and the like.

Although not particularly limited, the film thickness of the hole transport layer 3 is preferably 50 µm or less, more preferably 1 nm to 10 µm, still more preferably 5 nm to 5 µm, and still more preferably 10 nm to 1 µm. In addition, the film thickness of the hole transport layer 3 corresponds to an average distance between the second electrode 2 and the surface of the photosensitive layer 13, and can be measured by observing a cross-section of the photoelectric conversion element by using a scanning electron microscope (SEM) and the like.

### [Second Electrode]

The second electrode 2 functions as a positive electrode in a solar cell. The second electrode 2 is not particularly limited as long as the second electrode 2 has conductivity. Typically, the second electrode 2 can be configured to have the same configuration as that of the conductive support 11. In a case where sufficient strength is maintained, the support 11a is not necessary.

It is preferable that the second electrode 2 has a structure having a high current-collection effect. At least one of the conductive support 11 or the second electrode 2 needs to be substantially transparent so that light reaches the photosensitive layer 13. In the solar cell of the invention, it is preferable that the conductive support 11 is transparent and solar light is incident from the support 11a side. In this case, it is more preferable that the second electrode 2 has a light-reflecting property.

Examples of a material used to form the second electrode 2 include metals such as platinum (Pt), gold (Au), nickel (Ni), copper (Cu), silver (Ag), indium (In), ruthenium (Ru), palladium (Pd), rhodium (Rh), iridium (Ir), osmium (Os), and aluminum (Al), the above-described conductive metal oxides, carbon materials, conductive polymers, and the like. The carbon materials may be conductive materials formed through bonding of carbon atoms, and examples thereof include fullerene, a carbon nanotube, graphite, graphene, and the like.

As the second electrode 2, a thin film (including a thin film obtained through vapor deposition) of a metal or a conductive metal oxide, or a glass substrate or a plastic substrate which has the thin film is preferable. As the glass substrate or the plastic substrate, glass including a gold or platinum thin film or glass on which platinum is vapor-deposited is preferable.

The film thickness of the second electrode 2 is not particularly limited, and is preferably 0.01 to 100 µm, more preferably 0.01 to 10 µm, and still more preferably 0.01 to 1 µm.

### [Other Configurations]

In the invention, a spacer or a separator can also be used instead of the blocking layer 14 or in combination with the blocking layer 14 so as to prevent the first electrode 1 and the second electrode 2 from coming into contact with each other. In addition, a hole blocking layer may be provided between the second electrode 2 and the hole transport layer 3.

### [Solar Cell]

The solar cell of the invention is constituted by using the photoelectric conversion element of the invention. For example, as illustrated in Fig. 1A, Fig. 2 to Fig. 4, the photoelectric conversion element 10 having a configuration, which is allowed to operate by the external circuit 6, can be used as the solar cell. As the external circuit 6 that is connected to the first electrode 1 (the conductive support 11) and the second electrode 2, a known circuit can be used without particular limitation.

For example, the invention is applicable to individual solar cells described in Non-Patent Literature 1, J. Am. Chem. Soc., 2009, 131(17), p. 6050 to 6051, and Science, 338, p. 643(2012).

It is preferable that a lateral surface of the solar cell of the invention is sealed with a polymer, an adhesive, and the like so as to prevent deterioration, evaporation, and the like in constituent substances.

### [Method of Manufacturing Photoelectric Conversion Element and Solar Cell]

The photoelectric conversion element and the solar cell of the invention can be manufactured in accordance with a known method, for example, a method described in Non-Patent Literature 1, J. Am. Chem. Soc., 2009, 131(17), p. 6050 to 6051, Science, 338, p. 643(2012), and the like.

Hereinafter, the method of manufacturing the photoelectric conversion element and the solar cell of the invention will be described in brief.

In the manufacturing method of the invention, first, at least one of the blocking layer 14, the porous layer 12, or the electron transport layer 15 is formed on a surface of the conductive support 11 according to the purpose.

For example, the blocking layer 14 can be formed by a method in which a dispersion, which contains the insulating substance or a precursor compound thereof, and the like, is applied to the surface of the conductive support 11, and the dispersion is baked, a spray pyrolysis method, and the like.

A material that forms the porous layer 12 is preferably used as fine particles, and more preferably a dispersion that contains the fine particles.

A method of forming the porous layer 12 is not particularly limited, and examples thereof include a wet-type method, a dry-type method, and other methods (for example, a method described in Chemical Review, Vol. 110, p. 6595 (published on 2010)). In these methods, it is preferable that the dispersion (paste) is applied to the surface of the conductive support 11 or the surface of the blocking layer 14 and then the dispersion is baked at a temperature 100°C to 800°C for ten minutes to ten hours, for example, in the air. According to this, it is possible to bring the fine particles into close contact with each other.

In a case where baking is performed a plurality of times, a temperature in baking except final baking (a baking temperature except for a final baking temperature) is preferably set to be lower than the temperature in the final firing (the final baking temperature). For example, in a case where titanium oxide paste is used, the baking temperature except for the final baking temperature can be set in a range of 50°C to 300°C. In addition, the final baking temperature can be set in a range of 100°C to 600°C to be higher than the baking temperature except for the final baking temperature. In a case where a glass support is used as the support 11a, the baking temperature is preferably 60°C to 500°C.

The amount of a porous material applied to form the porous layer 12 is appropriately set in correspondence with the film thickness of the porous layer 12, the number of times of coating, and the like, and there is no particular limitation thereto. For example, the amount of the porous material applied per surface area 1 m² of the conductive support 11 is preferably 0.5 to 500 g, and more preferably 5 to 100 g.

In a case where the electron transport layer 15 is provided, the layer each can be formed in the same manner as in the hole transport layer 3 to be described below.

### Subsequently, the photosensitive layer 13 is provided.

Examples of a method of providing the photosensitive layer 13 include a wet-type method and a dry-type method, and there is no particular limitation thereto. In the invention, the wet-type method is preferable, and for example, a method of bringing an arbitrary layer into contact with a light absorbing agent solution that contains a perovskite-type light absorbing agent is preferable. In the method, first, the light absorbing agent solution for forming the photosensitive layer is prepared. The light absorbing agent solution contains MX₂ and AX which are raw materials of the perovskite compound. Here, A, M, and X are the same as A, M, and X in Formula (I). In the light absorbing agent solution, a molar ratio between MX₂ and AX is appropriately adjusted in correspondence with the purpose. In a case of forming the perovskite compound as the light absorbing agent, the molar ratio between AX and MX₂ is preferably 1:1 to 10:1. The light absorbing agent solution can be prepared by mixing MX₂ and AX in a predetermined molar ratio and, preferably, by heating the resultant mixture. The formation liquid is typically a solution, but may be a suspension. Heating conditions are not particularly limited. A heating temperature is preferably 30°C to 200°C, and more preferably 70°C to 150°C. Heating time is preferably 0.5 to 100 hours, and more preferably 1 to 3 hours. As a solvent or a dispersion medium, the following solvent or dispersion medium can be used.

Then, the light absorbing agent solution, which is prepared, is brought into contact with a surface of a layer (in the photoelectric conversion element 10, a layer of any one of the porous layer 12, the blocking layer 14, and the electron transport layer 15) on which the photosensitive layer 13 is to be formed. Specifically, application of the light absorbing agent solution or immersion in the light absorbing agent solution is preferable. A contact temperature is preferably 5°C to 100°C, and immersion time is preferably 5 seconds to 24 hours and more preferably 20 seconds to 1 hour. In a case of drying the light absorbing agent solution that is applied, with regard to the drying, drying with heat is preferable, and drying is performed by heating the applied light absorbing agent solution typically at 20°C to 300°C, and preferably at 50°C to 170°C.

In addition, the photosensitive layer can also be formed in conformity to a method of synthesizing the perovskite compound.

In addition, another example of the method includes a method in which an AX solution that contains AX, and an MX₂ solution that contains MX₂ are each independently applied (including an immersion method), and are dried as necessary. In this method, an arbitrary solution may be applied in advance, but it is preferable that the MX₂ solution is applied in advance. A molar ratio between AX and MX₂, application conditions, and drying conditions in this method are the same as described above. AX or MX₂ may be vapor-deposited instead of application of the AX solution and the MX₂ solution.

Still another example of the method includes a dry-type method such as a vacuum deposition by using a compound or a mixture from which a solvent of the light absorbing agent solution is removed. For example, a method of simultaneously or sequentially vapor-depositing AX and MX₂ may be exemplified.

According to the methods and the like, the perovskite compound is formed on the surface of the porous layer 12, the blocking layer 14, or the electron transport layer 15 as the photosensitive layer.

The hole transport layer 3 is formed on the photosensitive layer 13 that is provided as described above.

The hole transport layer 3 can be formed through application and drying of a hole transporting material solution that contains a hole transporting material. In the hole transporting material solution, a concentration of the hole transporting material is preferably 0.1 to 1.0 M (mol/L) when considering that application properties are excellent, and in a case of providing the porous layer 12, the hole transporting material solution easily intrudes into pores of the porous layer 12.

After the hole transport layer 3 is formed, the second electrode 2 is formed, thereby manufacturing the photoelectric conversion element.

The film thicknesses of the respective layers can be adjusted by appropriately changing the concentrations of respective dispersion liquids or solutions and the number of times of application. For example, in a case where the photosensitive layers 13B and 13C having a large film thickness are provided, a light absorbing agent solution may be applied and dried a plurality of times.

The respective dispersion liquids and solutions described above may respectively contain additives such as a dispersion auxiliary agent and a surfactant as necessary.

Examples of the solvent or dispersion medium that is used in manufacturing of the photoelectric conversion element include a solvent described in JP2001-291534A, but the solvent or dispersion medium is not particularly limited thereto. In the invention, an organic solvent is preferable, and an alcohol solvent, an amide solvent, a nitrile solvent, a hydrocarbon solvent, a lactone solvent, a halogen solvent, and a mixed solvent of two or more kinds thereof are preferable. As the mixed solvent, a mixed solvent of the alcohol solvent and a solvent selected from the amide solvent, the nitrile solvent, and the hydrocarbon solvent is preferable. Specifically, methanol, ethanol, isopropanol, γ-butyrolactone, chlorobenzene, acetonitrile, N,N'-dimethylformamide (DMF), dimethylacetamide, and a mixed solvent thereof are preferable.

A method of applying the solutions or dispersants which form the respective layers is not particularly limited, and it is possible to use a known application method such as spin coating, extrusion die coating, blade coating, bar coating, screen printing, stencil printing, roll coating, curtain coating, spray coating, dip coating, an inkjet printing method, and an immersion method. Among these, spin coating, screen printing, and the like are preferable.

The photoelectric conversion element of the invention may be subjected to an efficiency stabilizing treatment such as annealing, light soaking, and being left as is in an oxygen atmosphere as necessary.

The photoelectric conversion element prepared as described above can be used as a solar cell after connecting the external circuit 6 to the first electrode 1 and the second electrode 2.

### Examples

The photoelectric conversion element 10A and the solar cell illustrated in Fig. 1A were manufactured in the following procedure. In a case where the film thickness of the photosensitive layer 13 is large, this case corresponds to the photoelectric conversion element 10B and the solar cell illustrated in Fig. 2.

### [Preparation of Conductive Support]

As the transparent electrode 11b, a fluorine-doped SnO₂ conductive film having a film thickness of 300 nm was formed on a glass substrate having a thickness of 2.2 mm as the support 11a, thereby preparing the conductive support 11.

### [Preparation of Solution for Blocking Layer]

15% by mass isopropanol solution (manufactured by Sigma-Aldrich Co. LLC) of titanium diisopropoxide bis(acetylacetonate) was diluted with 1-butanol, thereby preparing 0.02 M solution for a blocking layer.

### [Formation of Blocking Layer]

The blocking layer 14 formed from titanium oxide, which has a film thickness of 100 nm, was formed on the SnO₂ conductive film of the conductive support 11 by using the prepared 0.02 M solution for the blocking layer at 450°C in accordance with a spray pyrolysis method.

### [Preparation of Titanium Oxide Paste]

Ethyl cellulose, lauric acid, and terpineol were added to an ethanol dispersion liquid of anatase-type titanium oxide having an average particle size of 20 nm, thereby preparing titanium oxide paste.

### [Formation of Porous Layer]

The prepared titanium oxide paste was applied onto the blocking layer 14 with a screen printing method, and was baked. Application and baking of the titanium oxide paste were respectively performed twice. With regard to a baking temperature and baking time, first baking was performed at 130°C for 1 hour, and second baking was performed at 500°C for 1 hour. A baked body of the titanium oxide, which was obtained, was immersed in 40 mM TiCl₄ aqueous solution, and was heated at 60°C for 1 hour, and heating was continuously performed at 500°C for 30 minutes, thereby forming the porous layer 12 formed from TiO₂ in a film thickness of 250 nm.

### [Formation of Photosensitive Layer]

A photosensitive layer was formed on the porous layer 12, which was formed as described above, as follows, thereby preparing the first electrode 1.

40% by mass of methylamine-methanol solution and 47% by mass of hydrogen bromide aqueous solution (hydromromic acid) were stirred in a flask at 0°C for 2 hours, and were concentrated to obtain coarse CH₃NH₃Br. The obtained coarse CH₃NH₃Br was dissolved in ethanol and was recrystallized with diethylehter. A crystal that precipitated was filtered and collected, and was dried under reduced pressure at 60°C for 24 hours, thereby obtaining purified CH₃NH₃Br.

The purified CH₃NH₃Br and PbBr₂ were collected in a molar ratio of 3:1, and were stirred and mixed in dimethylformamide (DMF) at 60°C for 12 hours, and the resultant mixture was filtered with a polytetrafluoroethylene (PTFE) syringe filter, thereby preparing a light absorbing agent solution of 40% by mass.

The light absorbing agent solution that was prepared was applied onto the porous layer 12 by a spin coating method under conditions of 2000 rpm for 60 seconds, and the applied light absorbing agent solution was dried by using a hot plate at 100°C for 60 seconds, thereby forming the photosensitive layer 13A composed of the perovskite compound of CH₃NH₃PbBr₃.

A perovskite compound of CH₃NH₃PbI₃ was prepared by respectively changing CH₃NH₃Br and PbBr₂ in the material that was used in the above-described manufacturing method to CH₃NH₃I and PbI₂.

### [Formation of Hole Transport Layer of Comparative Example 1 by Using Hole Transporting Material in Non-Patent Literature 1]

180 mg of 2,2',7,7'-tetrakis-(N,N-di-p-methoxyphenylamine)9,9'-spirobifluorene [spiro-OMeTAD] was dissolved in 1 mL of chlorobenzene. 37.5 µL of acetonitrile solution prepared by dissolving 170 mg of lithium-bis(trifluoromethanesulfonyl) imide in 1 mL of acetonitrile, and 17.5 µL of t-butylpyridine (TBP) were added to the chlorobenzene solution and the resultant mixture was mixed, thereby preparing a hole transporting material solution. Then, the prepared solution for the hole transport layer was applied onto the photosensitive layer in accordance with a spin coating method, and was dried, thereby forming a solid hole transport layer having a film thickness of 100 nm.

### [Formation of Hole Transport Layer of Examples 1 to 32 Using Hole Transporting Material of Invention, except for Examples 17, 24, 25 and 29]

A hole transport layer having a thickness of 100 nm was formed on the photosensitive layer by using each of hole transporting materials illustrated in the following Table 1 among chemical Formula 1 to Chemical Formula 51 described above as specific examples of the hole transporting material. At this time, a vapor deposition method was used.

### [Formation of Hole Transport Layer of Examples 33 to 38 Using Hole Transporting Material of Invention]

A hole transport layer having a thickness of 100 nm was formed on the photosensitive layer by using an application method in the same manner as in Comparative Example 1 except that each of the hole transporting materials of Chemical Formula 52 to Chemical Formula 57 described above as specific examples of the hole transporting material was used.

The film thickness of each layer was measured through observation with a scanning electron microscope (SEM) in accordance with the above-described method. In any example, the film thickness of the porous layer 12 was 250 nm, and a total film thickness of the porous layer 12 and the photosensitive layer 13A was 350 nm.

### [Preparation of Second Electrode]

Gold was vapor-deposited on the hole transport layer 3, thereby preparing the second electrode 2 having a film thickness of 0.3 µm.

In this manner, the photoelectric conversion elements 10 of Comparative Example 1 and Examples 1 to 38 were manufactured.

With respect to the photoelectric conversion elements 10, an absorption spectrum was measured in advance, and a value of absorbance at a predetermined wavelength was set to 100. Then, the absorption spectrum was measured again with respect to the photoelectric conversion elements 10 after passage of 45 hours under conditions of 30°C and 60 RH%, and moisture resistance was evaluated on the basis of a retention rate of the absorption at a predetermined wavelength. In addition, the absorption spectrum was measured at a portion, on which the second electrode (gold) was not vapor-deposited, of the photoelectric conversion elements 10. This configuration relates to evaluation using a phenomenon in which, in a case where a perovskite compound is decomposed, the absorbance decreases.

At this time, with regard to the hole transporting materials of chemical Formula 52 to Chemical Formula 57 which were used in Examples 33 to 38, overlapping between an absorption region of the hole transporting materials and an absorption region of the perovskite compound is great, and thus the following measurement was added so as to exclude an influence by the absorbance of the hole transporting material. That is, evaluation elements were separately manufactured. The evaluation elements have the same structure as in the photoelectric conversion elements 10 of Examples 33 to 38 except that the photosensitive layer (perovskite compound) is not included. In the photoelectric conversion elements 10 of Examples 33 to 38, and the evaluation elements which were additionally manufactured, the hole transport layers using the hole transporting materials of Chemical Formula 52 to Chemical Formula 57 were set to have the same film thickness. With respect to the respective evaluation elements, absorbance at a predetermined wavelength was measured before and after a test after the passage of 45 hours under conditions of 30°C and 60 RH%. The measured absorbance is obtained due to the hole transporting material of each of Chemical Formula 52 to Chemical Formula 57. In addition, before and after the test after the passage of time, moisture resistance was evaluated on the basis of the absorbance retention rate obtained by subtracting the absorbance of the evaluation elements from the absorbance of the photoelectric conversion elements 10 of Examples 33 to 38.

Evaluation was performed in correspondence with a material of the photosensitive layer, that is, at a wavelength of 520 nm in a case of CH₃NH₃PbBr₃ and at a wavelength of 650 nm in a case of CH₃NH₃PbI₃. Evaluation criteria are as follows.
Retention rate is 92% or greater: A
Retention rate is equal to or greater than 88% and less than 92%: B+
Retention rate is equal to or greater than 83% and less than 88%: B
Retention rate is equal to or greater than 78% and less than 83%: C
Retention rate is equal to or greater than 70% and less than 78%: D
Retention rate is less than 70%: E

Results are illustrated in the following Table 1, where Examples 17, 24, 25 and 29 do not form part of the invention.

From the following Table 1, it can be seen that the photoelectric conversion elements of Examples 1 to 38 which use the hole transporting materials of the invention exhibit more satisfactory moisture resistance in comparison to the photoelectric conversion element of Comparative Example 1 that uses spiro-OMeTAD as the hole transporting material.

Particularly, in the photoelectric conversion elements of Examples 23, 26, 31, and 32 using compounds, which include a tertiary amino group at both terminals and the tertiary amino group has an alkyl portion having 8 or more carbon atoms, among the hole transporting materials of the invention, the moisture resistance was very excellent.

In addition, in the photoelectric conversion elements of Examples 14, 21, 22, 26, and 28 using compounds, which include at least one substituent group represented by Formula L-2', L-3', L-4', or L-6', among the hole transporting materials of the invention, excellent moisture resistance is exhibited, and thus it is considered that the substituent groups contribute to an improvement of the moisture resistance in the photoelectric conversion elements.

However, in the photoelectric conversion elements of Examples 17 and 29 (not forming part of the invention) using compounds, which include an anthracene ring as a partial structure, among the hole transporting materials, the degree of improvement of moisture resistance is lower in comparison to photoelectric conversion elements of other examples using compounds which include a benzene ring, a naphthalene ring, or a phenanthrene ring as a partial structure. Accordingly, it is preferable that the hole transporting materials of the invention do not include the anthracene ring as a partial structure. Similarly, in the photoelectric conversion elements of Examples 17 to 19 using compounds, which do not include linear alkyl having 8 or more carbon atoms or a branched alkyl portion having 4 or more carbon atoms, among the hole transporting materials of the invention, the degree of improvement of the moisture resistance is low, and thus it is preferable that the hole transporting materials of the invention include linear alkyl having 8 or more carbon atoms or a branched alkyl portion having 4 or more carbon atoms.

**[Table 1]**

| | Photosensitive layer | Hole transporting material | Moisture resistance |
|---|---|---|---|
| Comparative Example 1 | CH3NH3PbBr3 | spiro- MeOTAD | E |
| Present Invention 1 | CH3NH3PbBr3 | Chemical Formula 1 | C |
| Present Invention 2 | CH3NH3PbBr3 | Chemical Formula 2 | B |
| Present Invention 3 | CH3NH3PbBr3 | Chemical Formula 3 | B |
| Present Invention 4 | CH3NH3PbBr3 | Chemical Formula 4 | B |
| Present Invention 5 | CH3NH3PbBr3 | Chemical Formula 5 | B |
| Present Invention 6 | CH3NH3PbBr3 | Chemical Formula 6 | B |
| Present Invention 7 | CH3NH3PbBr3 | Chemical Formula 7 | B |
| Present Invention 8 | CH3NH3PbBr3 | Chemical Formula 8 | B |
| Present Invention 9 | CH3NH3PbBr3 | Chemical Formula 9 | B |
| Present Invention 10 | CH3NH3PbBr3 | Chemical Formula 10 | B |
| Present Invention 11 | CH3NH3PbBr3 | Chemical Formula 11 | B |
| Present Invention 12 | CH3NH3PbBr3 | Chemical Formula 12 | B |
| Present Invention 13 | CH3NH3PbBr3 | Chemical Formula 13 | B |
| Present Invention 14 | CH3NH3PbBr3 | Chemical Formula 14 | B+ |
| Present Invention 15 | CH3NH3PbBr3 | Chemical Formula 15 | B |
| Present Invention 16 | CH3NH3PbBr3 | Chemical Formula 16 | B |
| Present Invention 17 | CH3NH3PbBr3 | Chemical Formula 17 | D |
| Present Invention 18 | CH3NH3PbBr3 | Chemical Formula 18 | C |
| Present Invention 19 | CH3NH3PbBr3 | Chemical Formula 19 | C |
| Present Invention 20 | CH3NH3PbBr3 | Chemical Formula 20 | B |
| Present Invention 21 | CH3NH3PbBr3 | Chemical Formula 21 | B+ |
| Present Invention 22 | CH3NH3PbBr3 | Chemical Formula 22 | B+ |
| Present Invention 23 | CH3NH3PbBr3 | Chemical Formula 23 | A |
| Present Invention 24 | CH3NH3PbBr3 | Chemical Formula 24 | B |
| Present Invention 25 | CH3NH3PbBr3 | Chemical Formula 25 | B |
| Present Invention 26 | CH3NH3PbBr3 | Chemical Formula 26 | B+ |
| Present Invention 27 | CH3NH3PbBr3 | Chemical Formula 27 | C |
| Present Invention 28 | CH3NH3PbBr3 | Chemical Formula 28 | B+ |
| Present Invention 29 | CH3NH3PbBr3 | Chemical Formula 29 | D |
| Present Invention 30 | CH3NH3PbI3 | Chemical Formula 22 | B+ |
| Present Invention 31 | CH3NH3PbBr3 | Chemical Formula 50 | A |
| Present Invention 32 | CH3NH3PbBr3 | Chemical Formula 51 | A |
| Present Invention 33 | CH3NH3PbBr3 | Chemical Formula 52 | B |
| Present Invention 34 | CH3NH3PbBr3 | Chemical Formula 53 | B |
| Present Invention 35 | CH3NH3PbBr3 | Chemical Formula 54 | B |
| Present Invention 36 | CH3NH3PbBr3 | Chemical Formula 55 | B |
| Present Invention 37 | CH3NH3PbBr3 | Chemical Formula 56 | C |
| Present Invention 38 | CH3NH3PbBr3 | Chemical Formula 57 | C |

Furthermore, a battery performance was measured with respect to specimens, which were separately manufactured, by using the same method as described above, it was confirmed that the specimens function as a solar cell when considering that a current flowed in any of the specimens.

## Claims

1. A photoelectric conversion element, comprising:
a first electrode that includes a photosensitive layer, which includes a light absorbing agent, on a conductive support; and
a second electrode that is opposite to the first electrode,
wherein the light absorbing agent includes a compound having a perovskite-type crystal structure that includes a cation of an element of Group 1 in the periodic table or a cationic organic group A, a cation of a metal atom M other than the element of Group 1 in the periodic table, and an anion of an anionic atom or atomic group X,
a hole transport layer, which includes a hole transporting material, is provided between the first electrode and the second electrode,
**characterised in that** the hole transporting material includes at least one kind of compound represented by any one of Formula 1 to Formula 16, including a condensed polycyclic aromatic group having a number of rings of 4 or greater,
at least two rings in the condensed polycyclic aromatic group are hetero rings including at least one atom selected from the group consisting of a sulfur atom, a nitrogen atom, a selenium atom, and an oxygen atom, and
the condensed polycyclic aromatic group includes at least one structure selected from the group consisting of a benzene ring, a naphthalene ring, an anthracene ring, and a phenanthrene ring as a partial structure 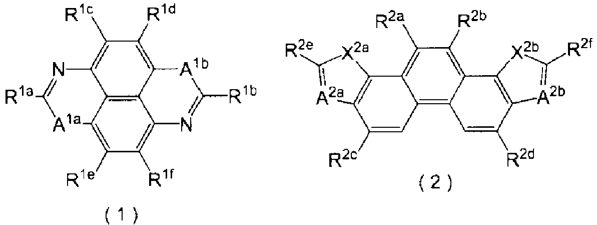 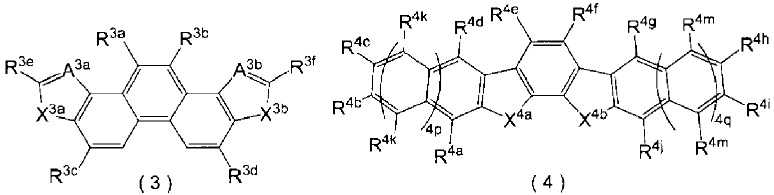 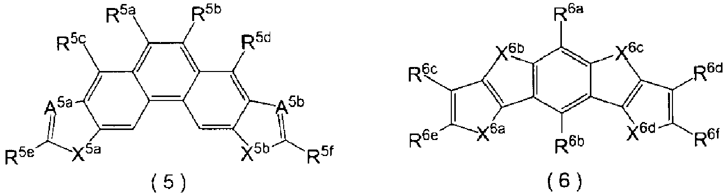 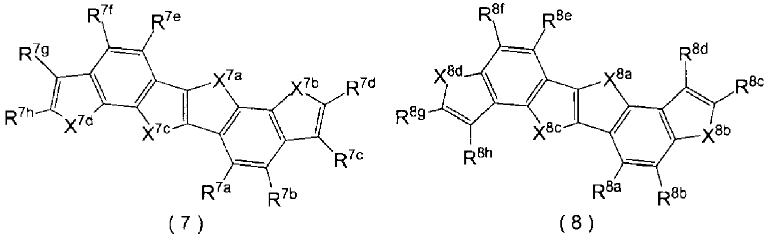 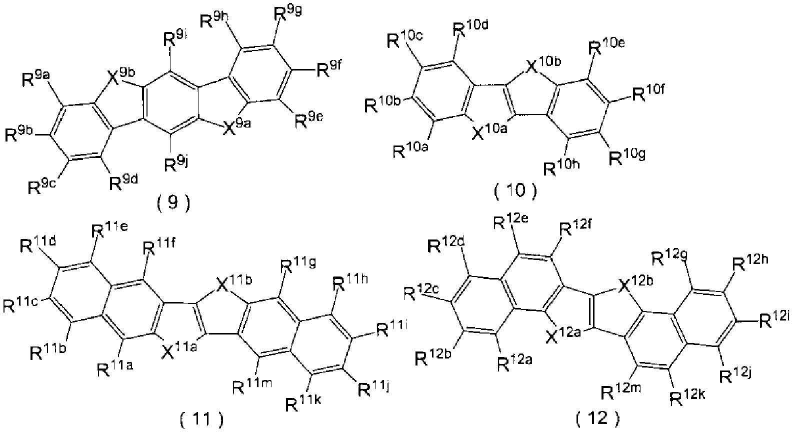 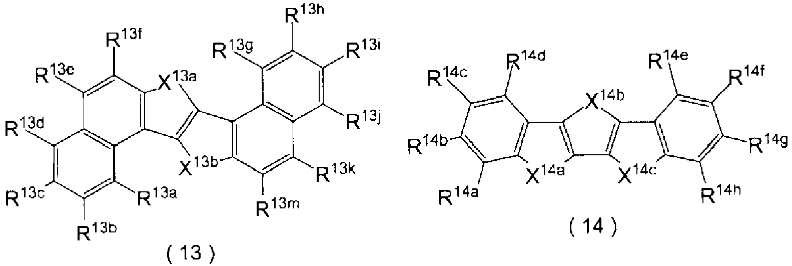 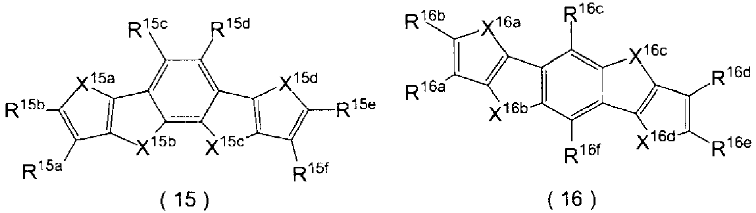
in Formula 1, A^{1a} and A^{1b} each independently represent a S atom, an O atom, or a Se atom, and R^{1a} to R^{1f} each independently represent a hydrogen atom or a substituent group,
in Formula 2, X^{2a} and X^{2b} each independently represent NR²ⁱ, an O atom, or a S atom, A^{2a} represents CR^{2g} or a N atom, A^{2b} represents CR^{2h} or a N atom, and R^{2a} to R²ⁱ each independently represent a hydrogen atom or a substituent group,
in Formula 3, X^{3a} and X^{3b} each independently represent a S atom, an O atom, or NR^{3g}, A^{3a} and A^{3b} each independently represent CR^{3h} or a N atom, R^{3a} to R^{3h} each independently represent a hydrogen atom or a substituent group,
in Formula 4, X^{4a} and X^{4b} each independently represent an O atom, a S atom, or a Se atom, 4p and 4q each independently represent an integer of 0 to 2, and R^{4a} to R^{4j}, R^{4k}, and R^{4m} each independently represent a hydrogen atom or a substituent group,
in Formula 5, X^{5a} and X^{5b} each independently represent NR⁵ⁱ, an O atom, or a S atom, A^{5a} represents CR^{5g} or a N atom, A^{5b} represents CR^{5h} or a N atom, and R^{5a} to R⁵ⁱ each independently represent a hydrogen atom or a substituent group,
in Formula 6, X^{6a} to X^{6d} each independently represent NR^{6g}, an O atom, or a S atom, and R^{6a} to R^{6g} each independently represent a hydrogen atom or a substituent group,
in Formula 7, X^{7a} and X^{7c} each independently represent a S atom, an O atom, a Se atom, or NR⁷ⁱ, X^{7b} and X^{7d} each independently represent a S atom, an O atom, or a Se atom, and R^{7a} to R⁷ⁱ each independently represent a hydrogen atom or a substituent group,
in Formula 8, X^{8a} and X^{8c} each independently represent a S atom, an O atom, a Se atom, or NR⁸ⁱ, X^{8b} and X^{8d} each independently represent a S atom, an O atom, or a Se atom, and R^{8a} to R⁸ⁱ each independently represent a hydrogen atom or a substituent group,
in Formula 9, X^{9a} and X^{9b} each independently represent an O atom, a S atom, or a Se atom, and R^{9A} to R^{9j} each independently represent a hydrogen atom or a substituent group,
in Formula 10, X^{10a} and X^{10b} each independently represent a S atom, an O atom, a Se atom, or NR¹⁰ⁱ, and R^{10a} to R¹⁰ⁱ each independently represent a hydrogen atom or a substituent group,
in Formula 11, X^{11a} and X^{11b} each independently represent a S atom, an O atom, a Se atom, or NR¹¹ⁿ, and R^{11a} to R^{11k}, R^{11m}, and R¹¹ⁿ each independently represent a hydrogen atom or a substituent group,
in Formula 12, X^{12a} and X^{12b} each independently represent a S atom, an O atom, a Se atom, or NR¹²ⁿ, and R^{12a} to R^{12k}, R^{12m}, and R¹²ⁿ each independently represent a hydrogen atom or a substituent group,
in Formula 13, X^{13a} and X^{13b} each independently represent a S atom, an O atom, a Se atom, or NR¹³ⁿ, and R^{13a} to R^{13k}, R^{13m}, and R¹³ⁿ each independently represent a hydrogen atom or a substituent group,
in Formula 14, X^{14a} to X^{14c} each independently represent a S atom, an O atom, a Se atom, or NR¹⁴ⁱ, and R^{14a} to R¹⁴ⁱ each independently represent a hydrogen atom or a substituent group,
in Formula 15, X^{15a} to X^{15d} each independently represent a S atom, an O atom, a Se atom, or NR^{15g}, and R^{15a} to R^{15g} each independently represent a hydrogen atom or a substituent group, and
in Formula 16, X^{16a} to X^{16d} each independently represent a S atom, an O atom, a Se atom, or NR^{16g}, and R^{16a} to R^{16g} each independently represent a hydrogen atom or a substituent group, and
wherein at least one of R^{1a} to R^{1f} in Formula 1, at least one of R^{2a} to R²ⁱ in Formula 2, at least one of R^{3a} to R^{3h} in Formula 3, at least one of R^{4a} to R^{4j}, R^{4k}, and R^{4m} in Formula 4, at least one of R^{5a} to R⁵ⁱ in Formula 5, at least one of R^{6a} to R^{6g} in Formula 6, at least one of R^{7a} to R⁷ⁱ in Formula 7, at least one of R^{8a} to R⁸ⁱ in Formula 8, at least one of R^{9a} to R^{9j} in Formula 9, at least one of R^{10a} to R^{10h} in Formula 10, at least one of R^{11a} to R^{11k}, R^{11m}, and R¹¹ⁿ in Formula 11, at least one of R^{12a} to R^{12k}, R^{12m}, and R¹²ⁿ in Formula 12, at least one of R^{13a} to R^{13k}, R^{13m}, and R¹³ⁿ in Formula 13, at least one of R^{14a} to R¹⁴ⁱ in Formula 14, at least one of R^{15a} to R^{15g} in Formula 15, and at least one of R^{16a} to R^{16g} in Formula 16 are groups represented by the following Formula W,
-L^{w}-R^{w} (W)
in Formula W, L^{w} represents a divalent linking group represented by any one of the following Formulae L-1 to L-25, or a divalent linking group in which two or more divalent linking groups each being represented by any one of the following Formulae L-1 to L-25 are bonded to each other, and R^{w} represents a hydrogen atom or a substituent group, 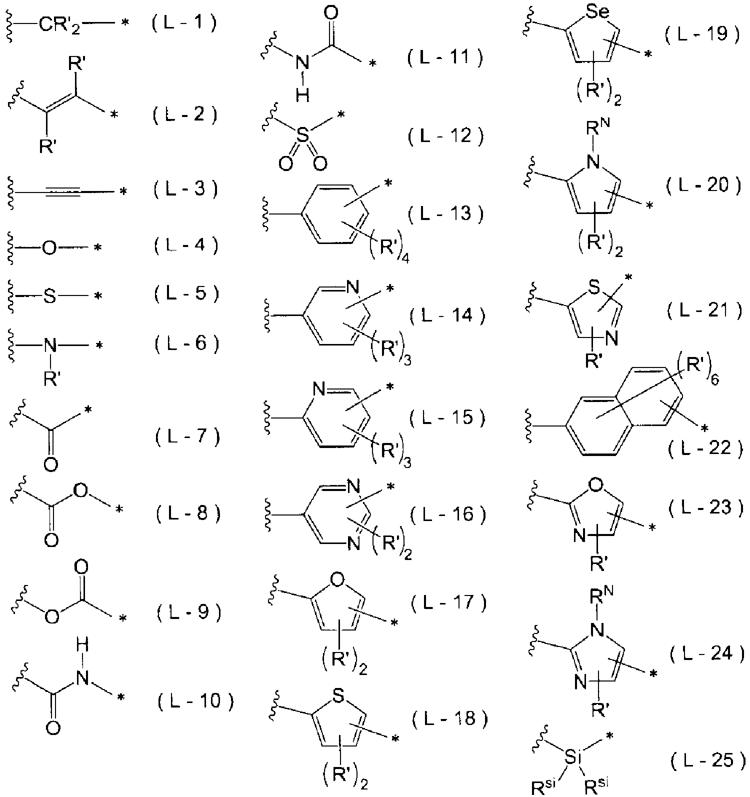 in Formulae L-1 to L-25, * represents a bonding position with R^{w}, a wavy-line portion represents another bonding position, R's in Formula L-1, Formula L-2, Formula L-6, and Formula L-13 to Formula L-24 each independently represent a hydrogen atom or a substituent group, R^{N}s represent a hydrogen atom or a substituent group, and R^{si}s each independently represent a hydrogen atom, an alkyl group, an alkenyl group, or an alkynyl group.

2. The photoelectric conversion element according to claim 1,
wherein the hole transporting material includes at least one structure selected from the group consisting of a benzene ring, a naphthalene ring, and a phenanthrene ring as the partial structure.

3. The photoelectric conversion element according to claim 1 or 2,
wherein the number of rings of the condensed polycyclic aromatic group is 4 to 6.

4. The photoelectric conversion element according to any one of claims 1 to 3,
wherein at least two hetero rings, which constitute the condensed polycyclic aromatic group, respectively include one hetero atom.

5. The photoelectric conversion element according to any one of claims 1 to 4,
wherein the hole transporting material includes at least one substituent group represented by the following Formula L-2', L-3', L-4', or L-6', 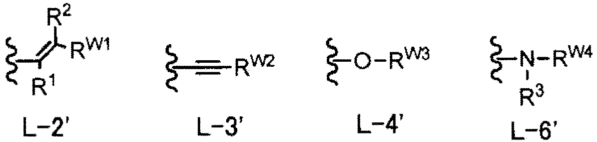 in Formula, R¹ to R³, and R^{W1} to R^{W4} represent a hydrogen atom, an alkyl group, an aryl group, or a heteroaryl group.

6. The photoelectric conversion element according to claim 5,
wherein the hole transporting material includes a substituent group that is represented by Formula L-2' or L-6'.

7. The photoelectric conversion element according to any one of claims 1 to 6,
wherein the hole transporting material includes a linear alkyl portion having 8 or more carbon atoms, or a branched alkyl portion having 4 or more carbon atoms.

8. A solar cell, comprising:
the photoelectric conversion element according to any one of claims 1 to 7.

## Patentansprüche

1. Photoelektrisches Umwandlungselement, umfassend:
eine erste Elektrode, die eine lichtempfindliche Schicht einschliesst, die ein Lichtabsorptionsmittel auf einem leitfähigen Träger einschliesst; und
eine zweite Elektrode gegenüber der ersten Elektrode,
wobei das Lichtabsorptionsmittel eine Verbindung mit einer Perovskit-Kristallstruktur einschliesst, die ein Kation eines Elements der Gruppe 1 des Periodensystems oder eine kationische organische Gruppe A, ein anderes Kation eines Metallatoms M als das Element der Gruppe 1 des Periodensystems und ein Anion eines anionischen Atoms oder einer Atomgruppe X einschliesst,
eine Lochtransportschicht, die ein Lochtransportmaterial einschliesst, das zwischen der ersten Elektrode und der zweiten Elektrode vorhanden ist,
**dadurch gekennzeichnet, dass**
das Lochtransportmaterial zumindest eine Art von Verbindungen, dargestellt durch irgendeine der Formel 1 bis Formel 16, einschliesslich einer kondensierten polycyclischen aromatischen Gruppe mit einer Ringzahl von 4 oder grösser, einschliesst,
zumindest zwei Ringe in der kondensierten polycyclischen aromatischen Gruppe Heteroringe sind, die zumindest ein Atom, ausgewählt aus der Gruppe bestehend aus einem Schwefelatom, einem Stickstoffatom, einem Selenatom und einem Sauerstoffatom, einschliessen, und
die kondensierte polycyclische aromatische Gruppe zumindest eine Struktur, ausgewählt aus der Gruppe bestehend aus einem Benzolring, einem Naphthalinring, einem Anthracenring und einem Phenanthrenring, als Teilstruktur einschliesst 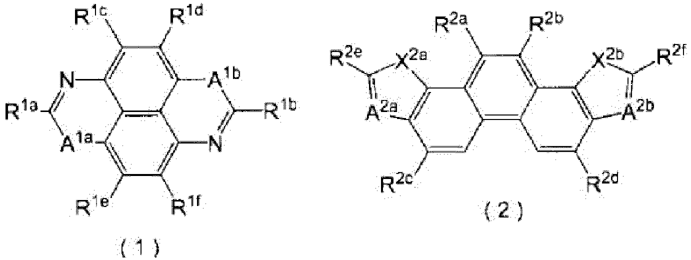 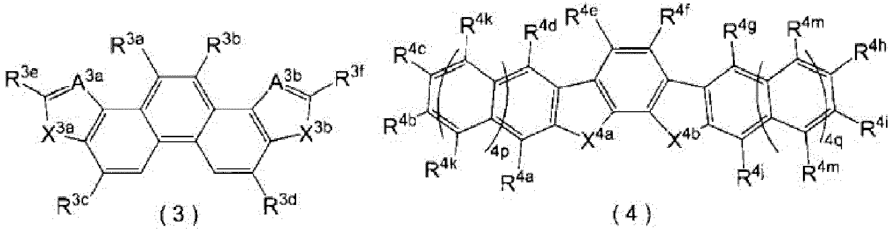 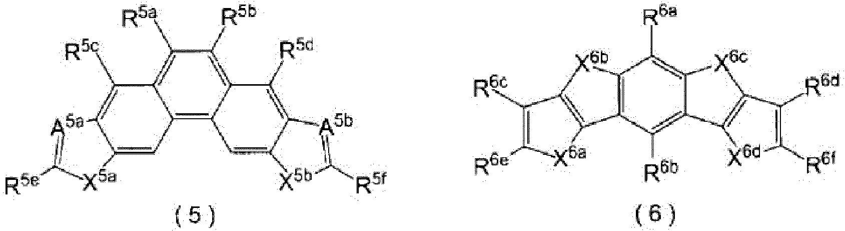 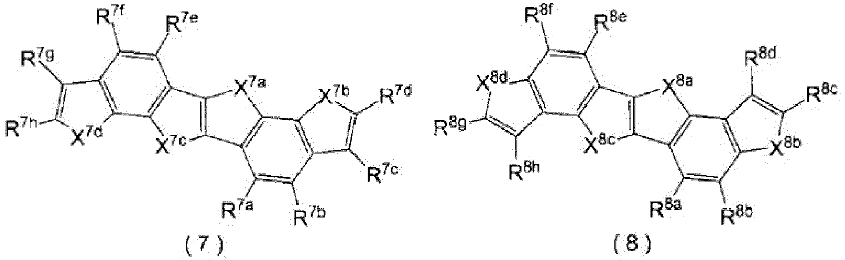 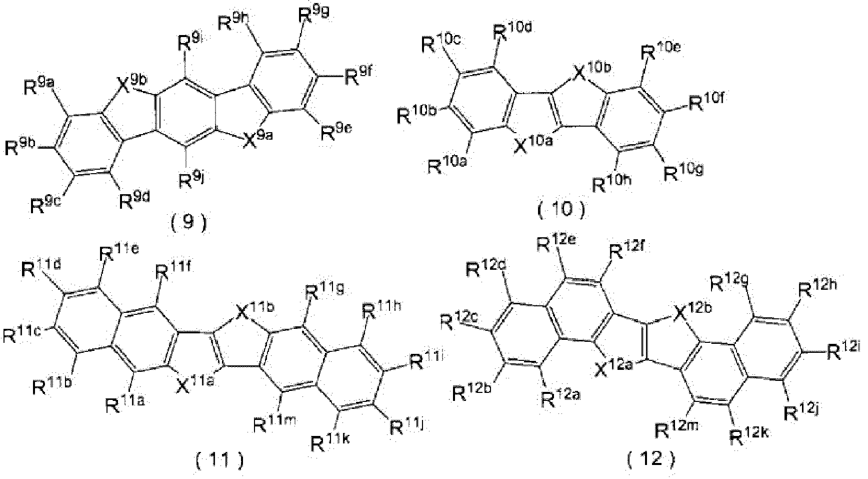 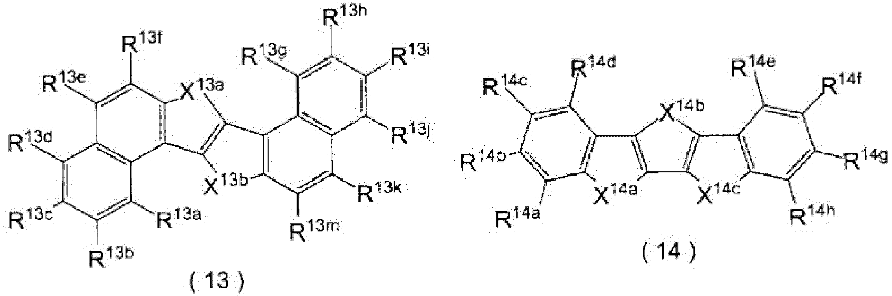 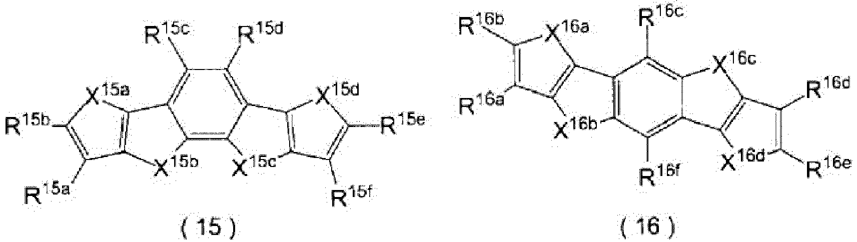
in der Formel 1 stellen A^{1a} und A^{1b} jeweils unabhängig ein S-Atom, ein O-Atom oder ein Se-Atom dar und R^{1a} bis R^{1f} stellen jeweils unabhängig ein Wasserstoffatom oder eine Substituentengruppe dar,
in der Formel 2 stellen X^{2a} und X^{2b} jeweils unabhängig NR²¹, ein O-Atom oder ein S-Atom dar, A^{2a} stellt CR^{2g} oder ein N-Atom dar, A^{2b} stellt CR^{2h} oder ein N-Atom dar und R^{2a} bis R²ⁱ stellen jeweils unabhängig ein Wasserstoffatom oder eine Substituentengruppe dar,
in der Formel 3 stellen X^{3a} und X^{3b} jeweils unabhängig ein S-Atom, ein O-Atom oder NR^{3g} dar, A^{3a} und A^{3b} stellen jeweils unabhängig CR^{3h} oder ein N-Atom dar, R^{3a} bis R^{3h} stellen jeweils unabhängig ein Wasserstoffatom oder eine Substituentengruppe dar,
in der Formel 4 stellen X^{4a} und X^{4b} jeweils unabhängig ein O-Atom, ein S-Atom oder ein Se-Atom dar, 4p und 4q stellen jeweils unabhängig eine ganze Zahl von 0 bis 2 dar und R^{4a} bis R^{4j}, R^{4k} und R^{4m} stellen jeweils unabhängig ein Wasserstoffatom oder eine Substituentengruppe dar,
in der Formel 5 stellen X^{5a} und X^{5b} jeweils unabhängig NR⁵ⁱ, ein O-Atom oder ein S-Atom dar, A^{5a} stellt CR^{5g} oder ein N-Atom dar, A^{5b} stellt CR^{5h} oder ein N-Atom dar und R^{5a} bis R⁵ⁱ stellen jeweils unabhängig ein Wasserstoffatom oder eine Substituentengruppe dar,
in der Formel 6 stellen X^{6a} bis X^{6d} jeweils unabhängig NR^{6g}, ein O-Atom oder ein S-Atom dar und R^{6a} bis R^{6g} stellen jeweils unabhängig ein Wasserstoffatom oder eine Substituentengruppe dar,
in der Formel 7 stellen X^{7a} und X^{7c} jeweils unabhängig ein S-Atom, ein O-Atom, ein Se-Atom oder NR⁷ⁱ dar, X^{7b} und X^{7d} stellen jeweils unabhängig ein S-Atom, ein O-Atom oder ein Se-Atom dar und R^{7a} bis R⁷ⁱ stellen jeweils unabhängig ein Wasserstoffatom oder eine Substituentengruppe dar,
in der Formel 8 stellen X^{8a} und X^{8c} jeweils unabhängig ein S-Atom, ein O-Atom, ein Se-Atom oder NR⁸ⁱ dar, X^{8b} und X^{8d} stellen jeweils unabhängig ein S-Atom, ein O-Atom oder ein Se-Atom dar und R^{8a} bis R⁸ⁱ stellen jeweils unabhängig ein Wasserstoffatom oder eine Substituentengruppe dar,
in der Formel 9 stellen X^{9a} und X^{9b} jeweils unabhängig ein O-Atom, ein S-Atom oder ein Se-Atom dar und R^{9a} bis R^{9j} stellen jeweils unabhängig ein Wasserstoffatom oder eine Substituentengruppe dar,
in der Formel 10 stellen X^{10a} und X^{10b} jeweils unabhängig ein S-Atom, ein O-Atom, ein Se-Atom oder NR¹⁰ⁱ dar und R^{10a} bis R¹⁰ⁱ stellen jeweils unabhängig ein Wasserstoffatom oder eine Substituentengruppe dar,
in der Formel 11 stellen X^{11a} und X^{11b} jeweils unabhängig ein S-Atom, ein O-Atom, ein Se-Atom oder NR¹¹ⁿ dar und R^{11a} bis R^{11k}, R^{11m} und R¹¹ⁿ stellen jeweils unabhängig ein Wasserstoffatom oder eine Substituentengruppe dar,
in der Formel 12 stellen X^{12a} und X^{12b} jeweils unabhängig ein S-Atom, ein O-Atom, ein Se-Atom oder NR¹²ⁿ dar und R^{12a} bis R^{12k}, R^{12m} und R¹²ⁿ stellen jeweils unabhängig ein Wasserstoffatom oder eine Substituentengruppe dar,
in der Formel 13 stellen X^{13a} und X^{13b} jeweils unabhängig ein S-Atom, ein O-Atom, ein Se-Atom oder NR¹³ⁿ dar und R^{13a} bis R^{13k}, R^{13m} und R¹³ⁿ stellen jeweils unabhängig ein Wasserstoffatom oder eine Substituentengruppe dar,
in der Formel 14 stellen X^{14a} bis X^{14c} jeweils unabhängig ein S-Atom, ein O-Atom, ein Se-Atom oder NR¹⁴ⁱ dar und R^{14a} bis R¹⁴ⁱ stellen jeweils unabhängig ein Wasserstoffatom oder eine Substituentengruppe dar,
in der Formel 15 stellen X^{15a} bis X^{15d} jeweils unabhängig ein S-Atom, ein O-Atom, ein Se-Atom oder NR^{15g} dar und R^{15a} bis R^{15g} stellen jeweils unabhängig ein Wasserstoffatom oder eine Substituentengruppe dar, und
in der Formel 16 stellen X^{16a} bis X^{16d} jeweils unabhängig ein S-Atom, ein O-Atom, ein Se-Atom oder NR^{16g} dar und R^{16a} bis R^{16g} stellen jeweils unabhängig ein Wasserstoffatom oder eine Substituentengruppe dar, und
wobei zumindest eines von R^{1a} bis R^{1f} in der Formel 1, zumindest eines von R^{2a} bis R²ⁱ in der Formel 2, zumindest eines von R^{3a} bis R^{3h} in der Formel 3, zumindest eines von R^{4a} bis R^{4j}, R^{4k} und R^{4m} in der Formel 4, zumindest eines von R^{5a} bis R⁵ⁱ in der Formel 5, zumindest eines von R^{6a} bis R^{6g} in der Formel 6, zumindest eines von R^{7a} bis R⁷ⁱ in der Formel 7, zumindest eines von R^{8a} bis R⁸ⁱ in der Formel 8, zumindest eines von R^{9a} bis R^{9j} in der Formel 9, zumindest eines von R^{10a} bis R^{10h} in der Formel 10, zumindest eines von R^{11a} bis R^{11k}, R^{11m} und R¹¹ⁿ in der Formel 11, zumindest eines von R^{12a} bis R^{12k}, R^{12m} und R¹²ⁿ in der Formel 12, zumindest eines von R^{13a} bis R^{13k}, R^{13m} und R¹³ⁿ in der Formel 13, zumindest eines von R^{14a} bis R¹⁴ⁱ in der Formel 14, zumindest eines von R^{15a} bis R^{15g} in der Formel 15 und zumindest eines von R^{16a} bis R^{16g} in der Formel 16 Gruppen der nachstehenden Formel W sind:
**-L^{W}-R^{W}** **(W)**
in der Formel W stellt L^{w} eine divalente Verknüpfungsgruppe, dargestellt durch irgendeine der nachstehenden Formeln L-1 bis L-25, oder eine divalente Verknüpfungsgruppe, in der zwei oder mehr divalente Verknüpfungsgruppen, jeweils dargestellt durch irgendeine der nachstehenden Formeln L-1 bis L-25, miteinander verbunden sind, dar und R^{w} stellt ein Wasserstoffatom oder eine Substituentengruppe dar, 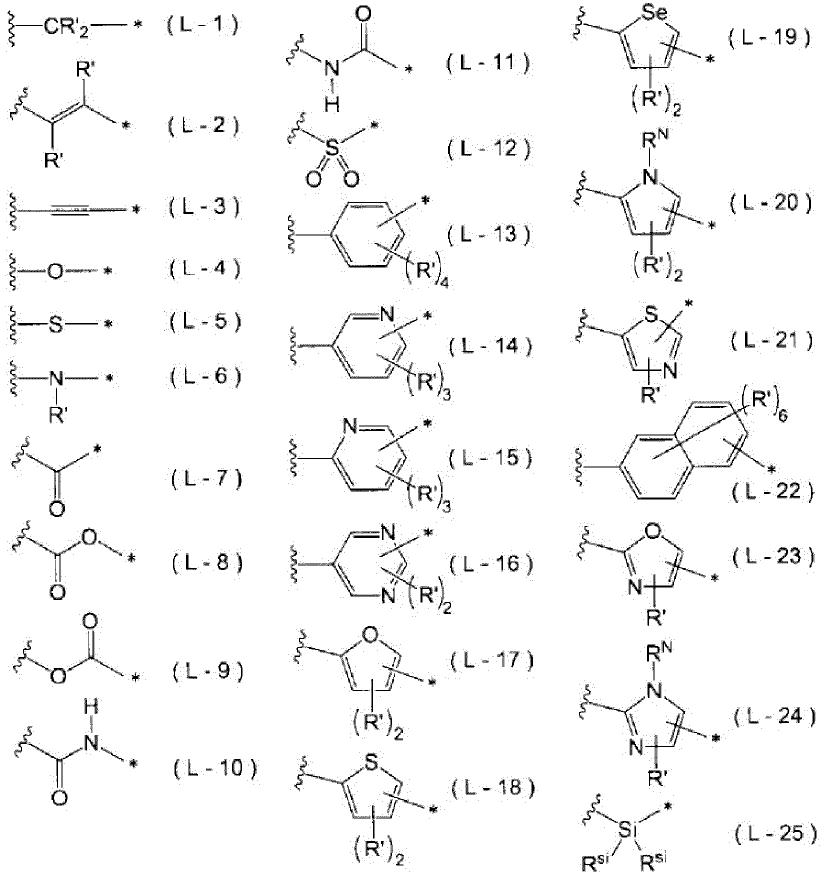 in den Formeln L-1 bis L-25 stellt * eine Bindungsstelle mit R^{w} dar, ein Wellenlinienabschnitt stellt eine andere Bindungsstelle dar, R' in der Formel L-1, Formel L-2, Formel L-6 und Formel L-13 bis Formel L-24 stellt jeweils unabhängig ein Wasserstoffatom oder eine Substituentengruppe dar, R^{N} stellt ein Wasserstoffatom oder eine Substituentengruppe dar und die R^{si} stellen jeweils unabhängig ein Wasserstoffatom, eine Alkylgruppe, eine Alkenylgruppe oder eine Alkinylgruppe dar.

2. Photoelektrisches Umwandlungselement gemäss Anspruch 1, wobei das Lochtransportmaterial zumindest eine Struktur, ausgewählt aus der Gruppe bestehend aus einem Benzolring, einem Naphthalinring und einem Phenanthrenring, als Teilstruktur einschliesst.

3. Photoelektrisches Umwandlungselement gemäss Anspruch 1 oder 2, wobei die Zahl der Ringe der kondensierten polycyclischen aromatischen Gruppe 4 bis 6 beträgt.

4. Photoelektrisches Umwandlungselement gemäss irgendeinem der Ansprüche 1 bis 3, wobei zumindest zwei Heteroringe, die die kondensierte polycyclische aromatische Gruppe bilden, ein Heteroatom einschliessen.

5. Photoelektrisches Umwandlungselement gemäss irgendeinem der Ansprüche 1 bis 4, wobei das Lochtransportmaterial zumindest eine Substituentengruppe der nachstehenden Formel L-2', L-3', L-4' oder L-6' einschliesst: 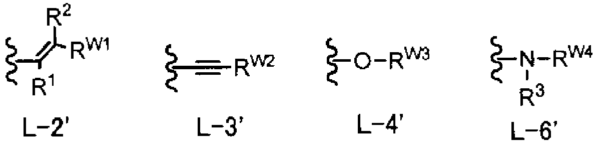 in der Formel stellen R¹ bis R³ und R^{W1} bis R^{W4} ein Wasserstoffatom, eine Alkylgruppe, eine Arylgruppe oder eine Heteroarylgruppe dar.

6. Photoelektrisches Umwandlungselement gemäss Anspruch 5, wobei das Lochtransportmaterial eine Substituentengruppe der Formel L-2' oder L-6' einschliesst.

7. Photoelektrisches Umwandlungselement gemäss irgendeinem der Ansprüche 1 bis 6, wobei das Lochtransportmaterial einen geradkettigen Alkylteil mit 8 oder mehr Kohlenstoffatomen oder einen verzweigten Alkylteil mit 4 oder mehr Kohlenstoffatomen einschliesst.

8. Solarzelle, die das photoelektrische Umwandlungselement gemäss irgendeinem der Ansprüche 1 bis 7 umfasst.

## Revendications

1. Élément de conversion photoélectrique, comprenant :
une première électrode qui inclut une couche photosensible, qui inclut un agent absorbant la lumière, sur un support conducteur; et
une seconde électrode qui est opposée à la première électrode,
dans lequel l'agent absorbant la lumière inclut un composé ayant une structure cristalline de type pérovskite qui inclut un cation d'un élément du Groupe 1 dans le tableau périodique ou un groupe organique cationique A, un cation d'un atome de métal M autre que l'élément du Groupe 1 dans le tableau périodique, et un anion d'un atome anionique ou d'un groupe atomique X,
une couche de transport de trous, qui inclut une matière de transport de trous, est prévue entre la première électrode et la seconde électrode,
**caractérisé en ce que**
la matière de transport de trous inclut au moins une sorte de composé représenté par n'importe laquelle de la Formule 1 à la Formule 16, incluant un groupe aromatique polycyclique condensé ayant un nombre d'anneaux de 4 ou plus,
au moins deux anneaux dans le groupe aromatique polycyclique condensé sont des hétéro-anneaux incluant au moins un atome sélectionné dans le groupe constitué par un atome de soufre, un atome d'azote, un atome de sélénium et un atome d'oxygène, et
le groupe aromatique polycyclique condensé inclut au moins une structure sélectionnée dans le groupe constitué par un anneau de benzène, un anneau de naphthalène, un anneau d'anthracène et un anneau de phénanthrène en tant que structure partielle 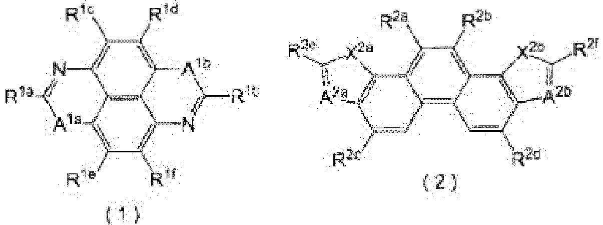 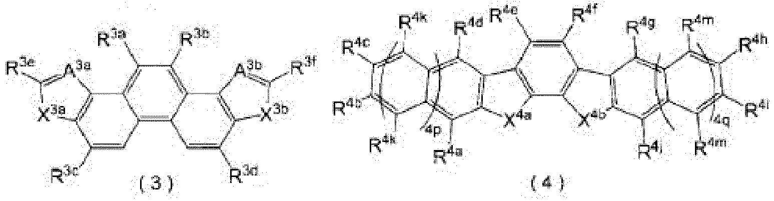 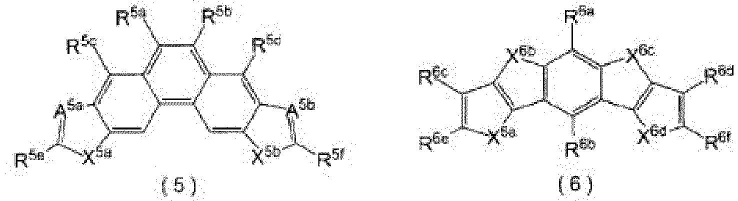 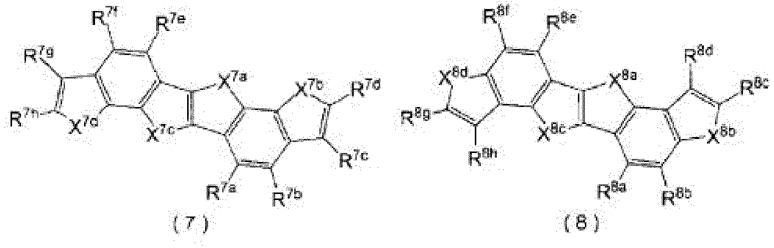 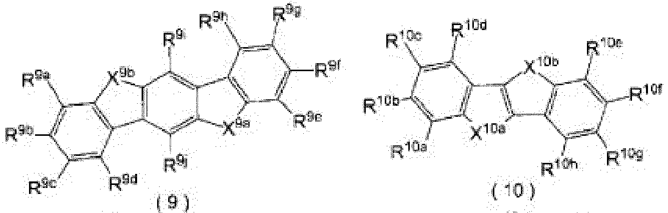 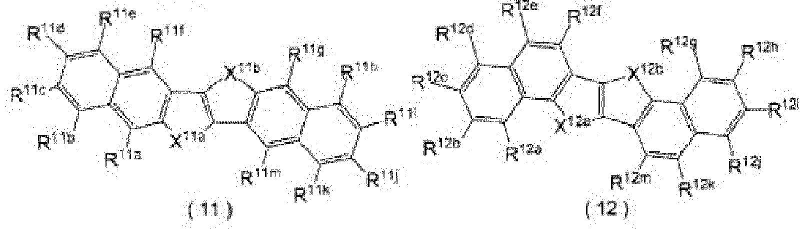 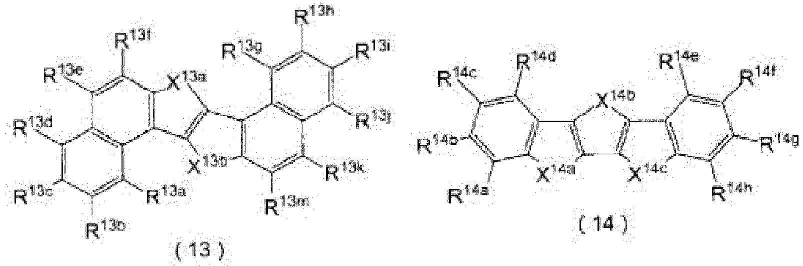 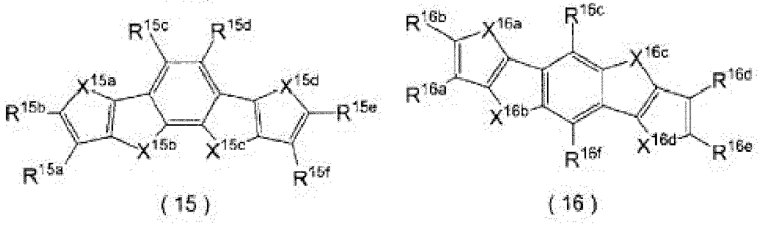
dans la Formule 1, A^{1a} et A^{1b} représentent chacun indépendamment un atome S, un atome O, ou un atome Se et R^{1a} à R^{1f} représentent chacun indépendamment un atome d'hydrogène ou un groupe substituant,
dans la Formule 2, X^{2a} et X^{2b} représentent chacun indépendamment NR²ⁱ, un atome O, ou un atome S, A^{2a} représente CR^{2g} ou un atome N, A^{2b} représente CR^{2h} ou un atome N et R^{2a} à R²ⁱ représentent chacun indépendamment un atome d'hydrogène ou un groupe substituant,
dans la Formule 3, X^{3a} et X^{3b} représentent chacun indépendamment un atome S, un atome O, ou NR^{3g}, A^{3a} et A^{3b} représentent chacun indépendamment CR^{3h} ou un atome N, R^{3a} à R^{3h} représentent chacun indépendamment un atome d'hydrogène ou un groupe substituant,
dans la Formule 4, X^{4a} et X^{4b} représentent chacun indépendamment un atome O, un atome S, ou un atome Se, 4p et 4q représentent chacun indépendamment un entier de 0 à 2 et R^{4a} à R^{4j}, R^{4k} et R^{4m} représentent chacun indépendamment un atome d'hydrogène ou un groupe substituant,
dans la Formule 5, X^{5a} et X^{5b} représentent chacun indépendamment NR⁵ⁱ, un atome O, ou un atome S, A^{5a} représente CR^{5g} ou un atome N, A^{5b} représente CR^{5h} ou un atome N et R^{5a} à R⁵ⁱ représentent chacun indépendamment un atome d'hydrogène ou un groupe substituant,
dans la Formule 6, X^{6a} à X^{6d} représentent chacun indépendamment NR^{6g}, un atome O, ou un atome S, et R^{6a} à R^{6g} représentent chacun indépendamment un atome d'hydrogène ou un groupe substituant,
dans la Formule 7, X^{7a} et X^{7c} représentent chacun indépendamment un atome S, un atome O, un atome Se, ou NR⁷ⁱ, X^{7b} et X^{7d} représentent chacun indépendamment un atome S, un atome O, ou un atome Se, et R^{7a} à R⁷ⁱ représentent chacun indépendamment un atome d'hydrogène ou un groupe substituant,
dans la Formule 8, X^{8a} et X^{8c} représentent chacun indépendamment un atome S, un atome O, un atome Se, ou NR⁸ⁱ, X^{8b} et X^{8d} représentent chacun indépendamment un atome S, un atome O, ou un atome Se, et R^{8a} à R⁸ⁱ représentent chacun indépendamment un atome d'hydrogène ou un groupe substituant,
dans la Formule 9, X^{9a} et X^{9b} représentent chacun indépendamment un atome O, un atome S, ou un atome Se, et R^{9a} à R^{9j} représentent chacun indépendamment un atome d'hydrogène ou un groupe substituant,
dans la Formule 10, X^{10a} et X^{10b} représentent chacun indépendamment un atome S, un atome O, un atome Se, ou NR¹⁰ⁱ, et R^{10a} à R¹⁰ⁱ représentent chacun indépendamment un atome d'hydrogène ou un groupe substituant,
dans la Formule 11, X^{11a} et X^{11b} représentent chacun indépendamment un atome S, un atome O, un atome Se, ou NR¹¹ⁿ, et R^{11a} à R^{11k}, R^{11m} et R¹¹ⁿ représentent chacun indépendamment un atome d'hydrogène ou un groupe substituant,
dans la Formule 12, X^{12a} et X^{12b} représentent chacun indépendamment un atome S, un atome O, un atome Se, ou NR¹²ⁿ, et R^{12a} à R^{12k}, R^{12m} et R¹²ⁿ représentent chacun indépendamment un atome d'hydrogène ou un groupe substituant,
dans la Formule 13, X^{13a} et X^{13b} représentent chacun indépendamment un atome S, un atome O, un atome Se, ou NR¹³ⁿ, et R^{13a} à R^{13k}, R^{13m} et R¹³ⁿ représentent chacun indépendamment un atome d'hydrogène ou un groupe substituant,
dans la Formule 14, X^{14a} à X^{14c} représentent chacun indépendamment un atome S, un atome O, un atome Se, ou NR¹⁴ⁱ, et R^{14a} à R¹⁴ⁱ représentent chacun indépendamment un atome d'hydrogène ou un groupe substituant,
dans la Formule 15, X^{15a} à X^{15d} représentent chacun indépendamment un atome S, un atome O, un atome Se, ou NR^{15g}, et R^{15a} à R^{15g} représentent chacun indépendamment un atome d'hydrogène ou un groupe substituant, et
dans la Formule 16, X^{16a} à X^{16d} représentent chacun indépendamment un atome S, un atome O, un atome Se, ou NR^{16g}, et R^{16a} à R^{16g} représentent chacun indépendamment un atome d'hydrogène ou un groupe substituant, et
dans lequel au moins un de R^{1a} à R^{1f} dans la Formule 1, au moins un de R^{2a} à R²ⁱ dans la Formule 2, au moins un de R^{3a} à R^{3h} dans la Formule 3, au moins un de R^{4a} à R^{4j}, R^{4k} et R^{4m} dans la Formule 4, au moins un de R^{5a} à R⁵ⁱ dans la Formule 5, au moins un de R^{6a} à R^{6g} dans la Formule 6, au moins un de R^{7a} à R⁷ⁱ dans la Formule 7, au moins un de R^{8a} à R⁸ⁱ dans la Formule 8, au moins un de R^{9a} à R^{9j} dans la Formule 9, au moins un de R^{10a} à R^{10h} dans la Formule 10, au moins un de R^{11a} à R^{11k}, R^{11m} et R¹¹ⁿ dans la Formule 11, au moins un de R^{12a} à R^{12k}, R^{12m} et R¹²ⁿ dans la Formule 12, au moins un de R^{13a} à R^{13K}, R^{13m} et R¹³ⁿ dans la Formule 13, au moins un de R^{14a} à R¹⁴ⁱ dans la Formule 14, au moins un de R^{15a} à R^{15g} dans la Formule 15, et au moins un de R^{16a} à R^{16g} dans la Formule 16 sont des groupes représentés par la Formule suivante W,
-L^{w}-R^{w} (W)
dans la Formule W, L^{w} représente un groupe de liaison divalent représenté par n'importe laquelle des Formules suivantes L-1 à L-25, ou un groupe de liaison divalent dans lequel deux ou plusieurs groupes de liaison divalents étant représentés chacun par n'importe laquelle des Formules suivantes L-1 à L-25 sont liés les uns aux autres, et R^{w} représente un atome d'hydrogène ou un groupe substituant, 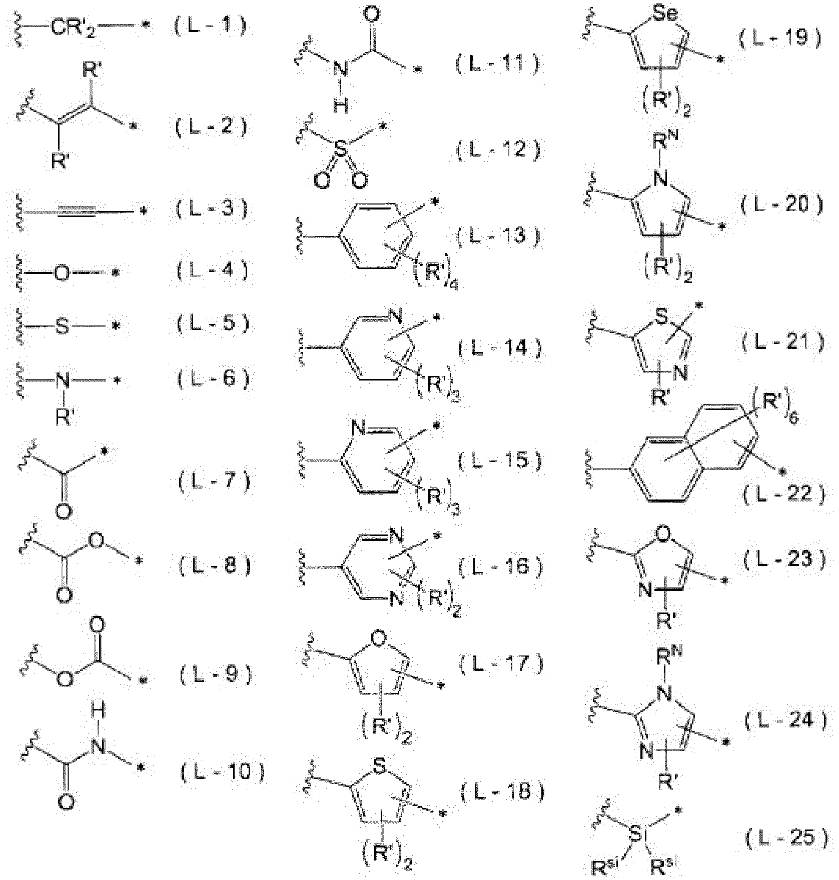 dans les Formules L-1 à L-25, * représente une position de liaison avec R^{w}, une portion de ligne ondulée représente une autre position de liaison, les R dans la Formule L-1, la Formule L-2, la Formule L-6 et la Formule L-13 à la Formule L-24 représentent chacun indépendamment un atome d'hydrogène ou un groupe substituant, les R^{N} représente un atome d'hydrogène ou un groupe substituant et les R^{si} représentent chacun indépendamment un atome d'hydrogène, un groupe alkyle, un groupe alcényle, ou un groupe alcynyle.

2. Élément de conversion photoélectrique selon la revendication 1,
dans lequel la matière de transport de trous inclut au moins une structure sélectionnée dans le groupe constitué par un anneau de benzène, un anneau de naphthalène et un anneau de phénanthrène en tant que structure partielle.

3. Élément de conversion photoélectrique selon la revendication 1 ou 2,
dans lequel le nombre d'anneaux du groupe aromatique polycyclique condensé est de 4 à 6.

4. Élément de conversion photoélectrique selon l'une quelconque des revendications 1 à 3,
dans lequel au moins deux hétéro-anneaux, qui constituent le groupe aromatique polycyclique condensé, incluent respectivement un hétéroatome.

5. Élément de conversion photoélectrique selon l'une quelconque des revendications 1 à 4,
dans lequel la matière de transport de trous inclut au moins un groupe substituant représenté par la Formule suivante L-2', L-3', L-4', ou L-6', 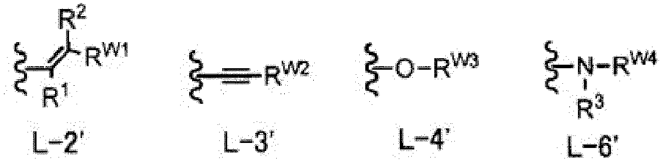 dans les Formules, R¹ à R³ et R^{W1} à R^{W4} représentent un atome d'hydrogène, un groupe alkyle, un groupe aryle, ou un groupe hétéroaryle.

6. Élément de conversion photoélectrique selon la revendication 5,
dans lequel la matière de transport de trous inclut un groupe substituant qui est représenté par la Formule L-2' ou L-6'.

7. Élément de conversion photoélectrique selon l'une quelconque des revendications 1 à 6,
dans lequel la matière de transport de trous inclut une portion alkyle linéaire ayant 8 atomes de carbone ou plus, ou une portion alkyle ramifiée ayant 4 atomes de carbone ou plus.

8. Cellule solaire, comprenant :
l'élément de conversion photoélectrique selon l'une quelconque des revendications 1 à 7.
